(19) Europäisches Patentamt
European Patent Office
Office européen des brevets

(11) Numéro de publication: **0 201 420**
**A1**

(12) # DEMANDE DE BREVET EUROPEEN

(21) Numéro de dépôt: **86400956.8**

(22) Date de dépôt: **30.04.86**

(51) Int. Cl.⁴: **C 07 K 9/00**, A 61 K 37/00, A 61 K 39/39

(30) Priorité: **30.04.85 FR 8506596**

(43) Date de publication de la demande: **12.11.86 Bulletin 86/46**

(84) Etats contractants désignés: **AT BE CH DE FR GB IT LI LU NL SE**

(71) Demandeur: **ANVAR Agence Nationale de Valorisation de la Recherche, 43, rue Caumartin, F-75436 Paris Cédex 09 (FR)**

(72) Inventeur: **Bernard, Jean-Marie 7 Square Couperin, Résidence du Parc St-Cyr, F-78330 Fontenay Le Fleury (FR)**
Inventeur: **Level, Michel, 24 rue Sibuet, F-75012 Paris (FR)**
Inventeur: **Lefrancier, Pierre, 46 allée de la Mare l'Oiseau Chevry 2, F-91190 Gif S/Yvette (FR)**
Inventeur: **Audibert, François, 44, rue Ybry, F-92200 Neuilly Sur Seine (FR)**
Inventeur: **Chedid, Louis, 18 rue Gaston Caillavet, F-75015 Paris (FR)**

(74) Mandataire: **Gutmann, Ernest et al, S.C. Ernest Gutmann - Yves Plasseraud 67, boulevard Haussmann, F-75008 Paris (FR)**

(54) **Glycopeptides du type 2-(2-acétamido-2-désoxy-3-O-D- glucopyranosyl)-alcanoyl-aminoacyl- nor Leucine ou dérivés de ces glycopeptides et compositions immunoadjuvantes les contenant.**

(57) L'invention concerne des composés choisis parmi des glycopeptides du type 2-(2-acétamido-2-désoxy-3-O-D-glucopyranosyl)-alcanoyl-aminoacyl-norLeucine sur lesquels sont, le cas échéant, greffés par couplage covalent, au moins un groupe porteur d'au moins un site antigénique. Ces composés, doués de propriétés immunoadjuvantes, peuvent être utilisés pour la constitution de compositions de vaccins.

EP 0 201 420 A1

86400956.8



Glycopeptides du type 2-(2-acétamido-2-désoxy-3-O-D- glucopyranosyl)-alcanoyl-aminoacyl- nor Leucine ou dérivés de ces glycopeptides et compositions immunoadjuvantes les contenant.

---

L'invention est relative à de nouveaux glycopeptides du type 2-(2-acétamido-2-désoxy-3-O-D-glucopyranosyl)-alcanoyl-aminoacyl-substitués, composés qui sont doués de propriétés biologiques et pharmacologiques de grande valeur. Plus particulièrement, l'invention concerne, parmi ces composés, ceux qui possèdent des propriétés immunorégulatrices, notamment d'adjuvants immunologiques non spécifiques, ces composés étant aptes, entre autres activités, à renforcer l'activité immunogène d'antigènes ou autres agents immunogènes naturels ou synthétiques. Par exemple, ces antigènes sont choisis parmi ceux qui sont aptes à promouvoir une activité immunoprotectrice in vivo contre des agents pathogènes.

De nombreux dérivés du type 2-(2-acétamido-2-désoxy-3-O-D-glucopyranosyl)-alcanoyl-peptides, souvent désignés par l'expression "muramyl-peptides" et les propriétés immunologiquement adjuvantes qu'ils possèdent sont bien connus. Ils font l'objet de nombreuses publications et de nombreux brevets, auxquels il sera d'ailleurs fait référence dans le cours de cette description. Il existe de nombreux analogues actifs, car de nombreuses modifications peuvent être réalisées en diverses parties de leur structure de base ; par exemple le noyau glucopyranosyle peut faire l'objet de substitutions diverses sans que les propriétés immunorégulatrices, notamment l'activité immunoadjuvante, soient perdues. Des observations semblables peuvent être faites lorsque le premier résidu aminoacyle, le plus souvent alanyle, du groupe peptidique du muramylpeptide

est remplacé par un autre résidu aminoacyle. Cependant, il a été considéré jusqu'à ce jour que la structure glutamyle du second aminoacyle de la chaîne peptidique était essentielle à la préservation des propriétés immunorégulatrices, plus particulièrement immunoadjuvantes, sans que soient pour autant interdites des variations aux niveaux des substitutions des groupes carboxyle de cette structure.

Cependant, les remplacements envisagés jusqu'à ce jour de la structure glutamyle par la structure de base dérivée d'un autre acide aminé naturel ont en général été accompagnés par une perte de l'activité.

L'invention fournit une nouvelle classe de composés qui échappe à cette règle générale. Les composés modifiés de l'invention conservent des propriétés immunoadjuvantes, d'un ordre de grandeur qui peut être semblable à celles de la N-acétyl-muramyl-L-alanyl-D-isoglutamine ou MDP, l'un des représentants les plus significatifs de la classe de muramyl-peptides. En outre, ils sont essentiellement dépourvus de propriétés pyrogènes, ce qui n'est pas le cas de tous les muramyl-peptides. Par ailleurs, ils sont métabolisés en dérivés eux-mêmes dépourvus de toute activité immunomodulatoire et de pyrogénicité, ce qui n'est pas le cas pour ce qui est de nombreux muramylpeptides qui s'hydrolysent facilement en MDP, connu pour présenter une certaine activité pyrogène. Enfin, ils sont sensiblement plus faciles à préparer que les muramylpeptides.

Les composés selon l'invention se distinguent pour l'essentiel des muramylpeptides ou de dérivés de muramylpeptides par la substitution d'un groupe ayant une structure de type <u>nor</u> Leucine au groupe glutamyle des muramylpeptides.

Plus particulièrement, l'invention concerne des composés choisis parmi les glycopeptides du type 2-(2-acétamido-2-désoxy-3-O-D-glucopyranosyl)-alcanoyl-aminoacyl-norLeucine et les dérivés desdits composés qui possèdent

des propriétés immunoadjuvantes. Parmi ces dérivés figurent ceux qui résultent du greffage sur le glycopeptide par couplage covalent d'au moins un groupe porteur d'au moins un site antigénique.

En particulier, le couplage est réalisé entre le glycopeptide du type 2-(2-acétamido-2-désoxy-3-O-glucopyranosyl)alcanoyl-aminoacyl-norLeucine comprenant au moins une fonction carboxyle, ester, alcool, sulfhydryle ou amine, apte à intervenir dans le couplage covalent, avec une fonction complémentaire correspondante de la molécule ou de l'agent porteur du site antigénique susdit.

Par "groupe porteur d'au moins un site antigénique", il faut entendre tout groupe dérivé d'un antigène à l'égard duquel une production in vivo d'anticorps est recherchée. Ce groupe porteur peut être constitué par un antigène de poids moléculaire élevé, naturel ou semi-synthétique contre lequel une protection in vivo est recherchée ou dont il convient d'accroître l'efficacité d'induction in vivo de la production d'anticorps orientés contre lui-même. Au titre des antigènes susceptibles d'être pris en considération dans le cadre de la présente demande de brevet, on mentionnera, par exemple, ceux qui font l'objet du brevet européen 3833 de la société CIBA GEIGY A.G. Parmi "les groupes porteurs d'au moins un site antigénique" figurent encore les haptènes porteurs d'un tel site antigénique et répondant à la définition qu'en donne la demande de brevet européen 89 290 déposée par l'Agence Nationale de Valorisation de la Recherche (ANVAR). Il ne s'agit donc pas seulement d'haptènes porteurs d'un site antigénique qui, lorsque ces haptènes sont couplés à un glycopeptide selon l'invention, leur confère la capacité d'induire in vivo des anticorps protecteurs contre des agents pathogènes déterminés. L'invention s'étend encore au couplage avec les glycopeptides d'autres haptènes, tels que ceux envisagés dans la

demande de brevet européen 89 290, par exemple des hormones.

Les composés préférés selon l'invention sont essentiellement caractérisés par la formule générale suivante:

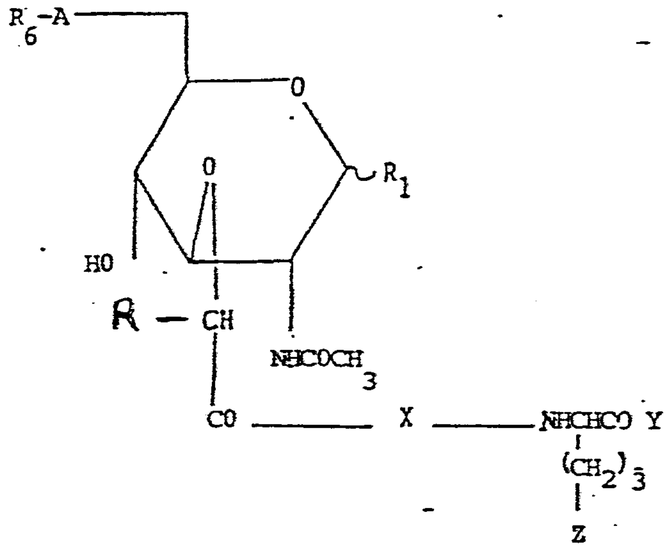

dans laquelle :

- $R_1$ est un groupe OH ou un groupe $O-C_6H_4-NH_2$ ou $O-(CH_2)_m-R_a$ m étant un nombre entier de 1 à 10, et $R_a$ étant une fonction amine, carboxyle, thiol, hydroxyle,
- A est de l'oxygène ou un groupe NH ;
- $R_6$ est de l'hydrogène ou un groupe B ou CO-B dans lequel B est un groupe ou une chaîne linéaire ou ramifiée pouvant comporter jusqu'à environ 100 atomes de carbone, ce groupe B étant encore éventuellement porteur de un ou plusieurs groupements fonctionnels, tels qu'amino, hydroxyle, carboxyle, carbonyle, alcoxyle, acyle, cyclopropane,
- R est un hydrogène ou un groupe alcoyle comprenant de 1 à 4 atomes de carbone, notamment méthyl ;
- X est un résidu alanyl, arginyl, lysyl, asparagyl, aspartyl, cystéinyl, glutaminyl, glutamyl, glycyl, histidyl, hydroxyprolyl, isoleucyl, leucyl, méthionyl, phénylalanyl, prolyl, séryl, thréonyl, tryptophanyl, ou valyl ;
- Y est un groupe OH, $NH_2$ ou, de préférence, $R_7$, $OR_7$ ou $NHR_7$,

$R_7$ étant un groupe hydrocarboné pouvant avoir de 1 à 20 atomes de carbone ou pouvant être un acide aminé tel qu'indiqué pour X, tel qu'alanyl, séryl, valyl et glycyl, ledit acide aminé étant libre, amidé ou estérifié, le groupe d'estérification pouvant comporter jusqu'à 4 atomes de carbone ;
- Z est un groupe alcoyle comprenant de 1 à 3 atomes de carbone, de préférence $-CH_3$ ou $-CH_2-CH_3$.

Les composés préférés sont ceux dans lesquels Y est un groupe $R_7$, $OR_7$ ou $NHR_7$, $R_7$ ayant la signification sus-indiquée, et dans lesquels le groupe NH-CH-COY est un dérivé

$$\begin{array}{c} NH-CH-COY \\ | \\ (CH_2)_3 \\ | \\ Z \end{array}$$

d'un groupe <u>nor</u> Leucine dextrogyre, plus particulièrement d'un groupe D-<u>nor</u> Leucine, dans lequel Z = $CH_3$.

A titre d'exemple et pour réaliser l'allongement de la chaîne latérale du deuxième acide aminé (Nle) :

$$\begin{array}{l} -NH-CH-CO \\ \quad\quad | \\ \quad (CH_2)_3-Z \end{array}$$

on peut avoir recours aux méthodes de synthèse et de résolution de la leucine, notamment celles décrites dans Chemistry of the amino acids. J.P. Greenstein, M. Winitz (J. Wiley Ed. New York, London) 1961.

Bien que le premier résidu aminoacyle X puisse être dextrogyre, il est préféré qu'il soit levogyre (sauf dans le cas du résidu glycyle).

Des significations particulièrement préférées pour X sont : alanyle, valyle, séryle ou glycyle.

Des classes préférées de composés selon l'invention sont celles dans lesquelles Y est $OR_7$, avec $R_7$ étant un groupe alcoyle comprenant de 1 à 10 atomes de carbone, plus particulièrement méthyle, n-butyle ou n-hexyle.

R est lui-même de préférence aussi constitué par un groupe méthyle. Dans une série de composés préférés de l'invention, $R_1$ est OH, A de l'oxygène et $R_6$ de l'hydrogène.

Ceci étant, d'autres composés préférés sont ceux

qui comportent des groupes fonctionnels, notamment du genre de ceux qui permettent leur couplage à d'autres constituants, par exemple, à des groupes porteurs d'au moins un site antigénique déterminé, et ce notamment par l'intermédiaire d'un groupe $R_a$ ou B.

Par exemple une classe avantageuse de composés utilisables pour de tels couplages sont ceux dans lesquels A est de l'oxygène et $R_6$ un groupe $-CO-(CH_2)_n-COOH$, tel que le groupe succinyle $-CO-(CH_2)_2-COOH$, ou le groupe $-CO-(CH_2)_n-NH_2$, tel que le groupe epsilon-amino-caproyle : $-CO-(CH_2)_6-NH_2$, n étant un nombre de 1 à 10.

Dans d'autres composés préférés de l'invention, les groupes $R_6$ contiennent seuls ou en combinaison avec les groupes qui viennent d'être mentionnés, des chaînes linéaires ou ramifiées, dont les maillons sont constitués de chaînes hydrocarbonées, dans lesquelles, le cas échéant, certains des atomes de carbone sont remplacés, de place en place, par des atomes d'azote et/ou d'oxygène, avec notamment pour conséquence la création de fonctions ester, éther, amido, imino, imido, etc.

Parmi les éléments avantageusement contenus dans les chaînes B, on mentionnera des résidus aminoacyle, notamment des chaînons contenant de 1 à 3 résidus aminoacyle successifs, par exemple, du type lysyle ou alanyle, (ceux-ci pouvant d'ailleurs être remplacés par tous autres aminoacyles du type indiqué à propos du groupe X), ou encore des éléments glycéryl : $-O-CH_2-CH(O)-CH_2-O-$ ou glycolyle : $-O-CH_2-CH_2-O-$.

D'autres groupes B préférés sont formés par des acides gras ou par des chaînes telles que ci-dessus définies, elles-mêmes porteuses de ramifications, par exemple par des groupes acylés. Des acides gras, préferés comprennent respectivement notamment de 14 à 30 atomes de carbone, par exemple les groupes palmitoyle.

D'autres groupes B préférés contiennent en particulier des éléments terminaux représentés par la formule :

$$-OCH_2-CHO(R_8)-CH_2O(R_9)$$

dans lesquels $R_8$ et $R_9$ sont des groupements acyle comprenant de 14 à 30, notamment, de 16 à 24 atomes de carbone.

La préparation des produits selon l'invention peut se faire à partir de l'acide muramique, de ses analogues ou de ses dérivés, lesquels ont en commun la structure ci-après appelée "structure muramique" :

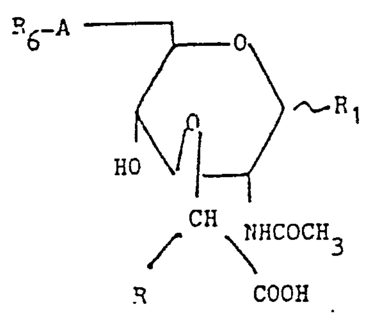

dans laquelle A, R, $R_1$ et $R_6$ ont les significations précédemment indiquées.

Des modes de préparations préférés des composés selon l'invention comprennent la fixation d'une chaîne peptidique de formule

$$X'-NH-CH[(CH_2)_3 - Z]- COY,$$

dans laquelle lorsque Y représente OH, $NH_2$, $OR_7$ ou $NHR_7$, X' représente X, et lorsque Y représente $R_7$, X' est un atome d'hydrogène, $R_7$, X et Z ayant les significations sus-indiquées, sur la structure muramique, préalablement couplée à un résidu aminoacide X dans le cas où Y représente $R_7$, par des méthodes traditionnelles dans le domaine de la synthèse des peptides et, le cas échéant, la fixation du groupe $-A-R_6$ sur la position 6 de la structure muramique.

Il est alors entendu que les fonctions portées par les partenaires de la réaction, et qui n'interviennent pas dans celle-ci, auront si besoin été protégées au préalable. Les fonctions protégées sont finalement libérées dans les phases terminales de la préparation des composés de l'invention. Les méthodes appliquées à la fabrication des muramylpeptides et décrites dans la littérature antérieure et en particulier dans les demandes de brevet français dont les références sont rappelées plus loin, peuvent également être appliquées à la production des composés selon l'invention.

S'il y a lieu, la substitution Y est avantageusement réalisée sur le groupe nor Leucine, avant la synthèse de la chaîne.

La synthèse de la chaîne formée par le résidu aminoacyle X et le groupe nor Leucine (ou homologue de celuici) est réalisée suivant des méthodes traditionnelles applicables aux synthèses de peptides. A titre d'exemple, on peut choisir les méthodes d'activation des carboxyles, comme la méthode des esters activés ou celle dite des anhydrides mixtes, ou bien celle utilisant un composé du type carbodiimide tel que le N,N'-dicyclohexylcarbodiimide ou des carbodiimides équivalents. On trouvera une revue des modes de synthèse peptidique traditionnels dans J.H. JONES, Chemistry and Industry, 723 (1974). On peut aussi se reporter aux demandes de brevets français déjà cités ou encore aux demandes suivantes : N° 75 29624, 76 06819, 76 06820, 76 06821, 76 21889, 77 02646, et aux articles de LEFRANCIER et al. (Int. J. Peptide Protein Res., 1977, 9, 249 et 1978, 11, 289 et J. Med. Chem. 25, 1982, 87).

La formation des dérivés estérifiés ou amidés

correspondant au groupe Y est obtenue de façon connue. On peut en particulier se reporter aux demandes de brevets français indiqués ci-dessus, et notamment aux demandes N° 76 06820, 76 06821, 76 21889 et 77 02646.

La synthèse du glycopeptide correspondant est alors complétée par le couplage avec la structure muramique de la chaîne aminoacyle-nor Leucine après libération préalable de la fonction amine N-terminale. Cette structure muramique aura elle-même été protégée au préalable. Elle sera par exemple constituée par l'acide alpha-O-benzyl-4,6-O-benzylidène-N-acétyl-muramique. Les dérivés glycopeptidiques sont finalement obtenus à l'état libre, après élimination (par hydrogénolyse par exemple) des groupements protecteurs.

On décrit ci-après des modes de préparations préférés de glycopeptides de formule générale :

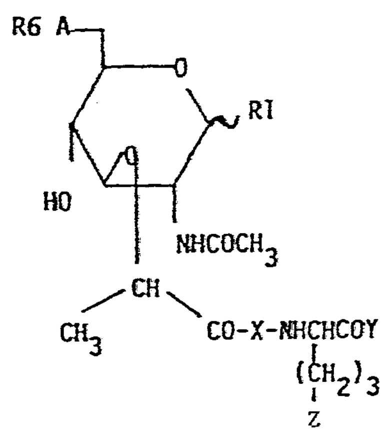

I) Cas dans lequel A est un oxygène

a) Y est un groupe OH, $NH_2$, $OR_7$ ou $NHR_7$.

Le produit de départ est un dérivé 4,6 benzylidène de l'acide muramique, $R_1$ représentant un radical benzyle glycoside (ou d'autres groupes glycosides), préparé comme décrit par Gross et Jeanloz (J. Org. Chem. 1967, 32, 2759). Pour obtenir un composé semblable dans lequel $R_1$ soit un groupement autre que oxybenzyle, on peut utiliser

la méthode de préparation d'α ou β-glycosides décrite dans ce même article, ou toute méthode décrite pour de telles préparations en chimie des oligosaccharides. Ces dérivés de l'acide muramique peuvent être couplés avec un dérivé dipeptidique de formule générale H-X-NH-CH-COY, préparés

$$\text{H-X-NH-}\underset{\underset{\displaystyle Z}{|}}{\underset{(CH_2)_3}{\overset{|}{\text{CH}}}}\text{-COY}$$

suivant des méthodes notamment décrites par Lefrancier et al. (Int. J. Peptide Protein Res 1977, 9, 249 ; 1978, 11, 289 ; 1979, 14, 437 ; J. Med. Chem. 1982, 25, 87). Les méthodes de couplage utilisées pour obtenir les dérivés glycopeptidiques sont également décrites dans les articles précédemment cités. Cependant, aussi bien dans la synthèse des dérivés dipeptidiques que dans celle des dérivés glycopeptidiques, toute méthode de couplage connue en synthèse peptidique peut être utilisée.

Les dérivés glycopeptidiques subissent une débenzylidénation sélective telle que décrite, par exemple, par Merser et al. (Biochem. Biophys. Res. Commun. 1975, 66, 1316).

L'acylation sélective de l'hydroxyle primaire, en position C-6 du résidu muramique ainsi libéré, peut alors se faire soit par action d'un anhydride d'un acide carboxylique, par exemple, dans le cas où $R_6$ est un résidu d'acide succinique, soit par couplage d'un acide carboxylique au moyen de toute méthode de conjugaison (formation d'une liaison ester) connue en synthèse peptidique, par exemple, dans le cas où $R_6$ est un résidu d'acide N-benzyloxycarbonyl-ε-aminocaproïque, ou bien un acide gras de nature simple (par exemple l'acide palmitique) ou complexe (par exemple acides mycoliques ou acide 1,2-dipalmitoylglycérique). Ces mêmes dérivés peuvent être également obtenus via une tosylation spécifique de l'hydroxyle en position C-6 du résidu muramyle, telle que décrite notamment

par Kusumoto et al. (Tetrahedron Letters, 1976, 47, 4237). Suivant le cas, notamment si $R_1$ est un groupement benzyle glycoside à éliminer, les glycopeptides sont finalement obtenus après hydrogénation catalytique.

b) <u>Y est un groupe $R_7$</u>

Le produit de départ est un dérivé 4,6-benzylidène de l'acide muramique décrit ci-dessus, couplé à un résidu aminoacide X, suivant la méthode notamment décrite par Lefrancier P, et Lederer E.in Progress in the Chemistry of Organic Products, ed.Herz W., Grisebach M. et Kirby G.W., p.1 (1981) - Springer - Verlag, Vienne. Ce dérivé peut être couplé avec un dérivé de formule générale

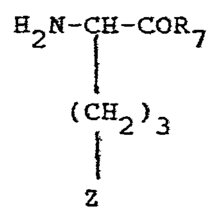

préparé suivant des méthodes notamment décrites dans les articles de Mukaiyama T. et al, J. AM.Chem.Soc., 95 (1973) 4763- 4765, et de Jonhson R.L. et al, Int.J. Pept. Prot. Res., 23 (1984) 581-590.

2) <u>Cas dans lequel A est un groupement NH</u>.

Cette variante a comme produit de départ un dérivé 6-azido de l'acide muramique, lui-même obtenu par débenzylidénation sélective du dérivé 4,6-benzylidène de l'acide muramique de départ, envisagé dans le cas précédent.

Ces dérivés 6-azido sont préparés comme décrit notamment par Hasegawa et al. (Agric. Biol. Chem. 1980, 44, 1309). Par la suite, les synthèses conduisant aux glycopeptides correspondant à la formule générale pour lesquels A est un groupement NH, sont semblables à celles conduisant aux glycopeptides correspondant à la formule générale pour lesquels A est un oxygène. En fait, ces synthèses comprennent un couplage avec un dérivé dipeptidique adéquat une hydrogénation catalytique transformant le groupement azido en groupement amino et finalement une acylation par les acides carboxyliques (notamment ceux cités dans la variante précédente).

Les composés ayant des propriétés immunoadjuvantes selon la présente invention comprennent les composés dérivant du couplage entre un glycopeptide de formule I ci-dessus, dans laquelle A, R, $R_6$, X, Y et Z sont tels que définis ci-dessus, un site particulièrement approprié à ce couplage se situant au niveau du groupe $R_1$, de préférence lorsque $R_1$ représente un groupe $O-(CH_2)_m-R_a$, m et $R_a$ étant tels que définis ci-dessus.

Les composés ayant des propriétés immunoadjuvantes selon la présente invention comprennent également les produits dérivant du couplage entre un glycopeptide de formule I ci-dessus, dans laquelle A, R, $R_6$, X, Y et Z sont tels que définis ci-dessus, $R_1$ est un groupe OH et $R_5$ est un

groupe B ou CO-B, B étant tel que défini ci-dessus. Dans ce cas, le site particulièrement approprié à ce couplage se situe au niveau du groupe B.

Il va de soi que le couplage peut s'effectuer de toute autre façon appropriée, dès lors que les propriétés immunoadjuvantes des conjugués obtenus seront conservées.

En particulier, les différentes variantes des techniques indiquées dans la demande de brevet européen 89 290 pour réaliser le couplage entre un haptène et un muramylpeptide peuvent être transposés dans des conditions semblables à la conjugaison entre les glycopeptides selon la présente invention et des haptènes semblables. Les mêmes observations s'étendent aux procédés de couplage covalent qui ont été désignés dans le brevet européen 3833, pour ce qui est de la réalisation de conjugaisons covalentes entre des antigènes et des muramylpeptides, ces procédés pouvant de la même manière être transposés à la réalisation de conjugaisons entre des antigènes et des glycopeptides conformes à la présente invention.

La description de ces techniques elles-mêmes doit être considérée comme incorporée à la rédaction du présent brevet pour autant que le contenu de ces documents antérieurs soit considéré comme nécessaire à la compréhension totale de la présente invention.

Il sera néanmoins rappelé que, de préférence, on aura recours à des procédés tel que celui décrit par FRANTZ et ROBERTSON dans Infect. and Immunity, 33, 193-198 (1981) ou celui décrit dans Applied and Environmental Microbiology, Octobre 1981, vol. 42, N° 4, 611-614 par E. KAUFFMAN en utilisant le peptide et la molécule porteuse appropriée. Ces divers procédés mettent en jeu des agents de couplage, particulièrement favorables dès lors qu'il s'agit de réaliser le couplage entre un antigène ou haptène et un glycopeptide conforme à l'invention, porteurs respectivement de

fonctions chimiques complémentaires telles que celles que l'on rencontre dans la chimie des peptides.

Dans la pratique, on utilisera avantageusement comme agent de couplage les composés suivants, cités à titre non limitatif : aldéhyde glutarique, chloroformiate d'éthyle, carbodiimides hydrosolubles [N-éthyl-N'(3-diméthylamino-propyl)carbodiimide, HCl], diisocyanates, bis-diazobenzidine, di- et tri-chloro-s-triazines, bromure de cyanogène, benzoquinone, ainsi que les agents de couplage mentionnés dans Scand. J. Immunol., 1978, vol. 8, p. 7-23 (AVRAMEAS, TERNYNCK, GUESDON).

On peut avoir recours à tout procédé de couplage faisant intervenir d'une part une ou plusieurs fonctions réactives du peptide et d'autre part, une ou plusieurs fonctions réactives des molécules supports. Avantageusement, il s'agit des fonctions carboxyle et amine, lesquelles peuvent donner lieu à une réaction de couplage en présence d'un agent de couplage du genre de ceux utilisés dans la synthèse des protéines, par exemple le 1-éthyl-3-(3-diméthylaminopropyl)-carbodiimide, le N-hydroxybenzotriazole, etc. On peut encore avoir recours à la glutaraldéhyde, notamment, lorsqu'il s'agit de relier entre eux des groupes aminés respectivement portés par le peptide et la molécule support.

D'autres caractéristiques de l'invention apparaîtront encore au cours de la description qui suit d'exemples préférés de glycopeptides conformes à la présente invention et de leur mode de préparation. On remarquera tout d'abord que, dans ce qui suit, l'on utilisera pour la désignation de ces glycopeptides des écritures abrégées semblables à celles couramment utilisées dans le domaine des muramyl-peptides. En particulier, le groupe 2-acétamido-2-déoxy-3-O (D-2-alcanoyl)D-glycopyranose ——————————
est ci-après désigné sous l'expression MurNAC, lorsque

R = $CH_3$ et sous l'expression nor MurNAc lorsque R = H.

D'autres abréviations utilisées (en sus des abréviations classiques pour les acides aminés classiques) ont les significations suivantes :

(3) Nle : norleucine
(4) Me : méthyle
(5) Bu : butyle
(6) Hex : hexyle
(7) BOC : t-butyl-oxycarbonyle
(8) Bzl : benzyle
(9) Bzi : benzylidène
(10) Su : succinyle
(11) DMF : diméthylformamide
(12) pts : para-toluène sulfonate

Dans ce qui suit, il sera également renvoyé aux références bibliographiques suivantes :


1. ANDERSON G.W., Zimmerman J.E. and Callahan F.M. (1964) J. Am. Chem. Soc. 86, 1839.
2. MERSER C., Sinaÿ Pand Adam A. (1975) Biochem. Biophys. Res. Comm. 66, 1316.
3. OSAWA T. et Jeanloz R.W. (1965) J. Org. Chem. 30, 488.
4. LEFRANCIER P., Derrien M, Lederman i, Nief F, Choay J and Lederer E. (1978). Int. J. Peptide Brotein Res. II 289.

Mur NAc-L-Ala-D-Nle-$NH_2$

BOC-L-Ala-D-Nle-OMe (I)

572 mg (2mmoles) de BOC-L-Ala-OSu (1) sont ajoutés à une solution, refroidie à 0°C, de 400 mg (2,2 mmoles) de D-Nle-OMe, HCl et de 0,24 ml (2,2 mmoles) de N-methylmorpholine dans 5 ml de DMF. Après une nuit, sous agitation, à température ambiante, le mélange réactionnel est évaporé à sec et le résidu obtenu repris dans l'acétate d'éthyle. La phase organique est successivement lavée avec une solution d'acide citrique 10 %, à l'eau, avec une solution 1N Na $HCO_3$ puis à l'eau ; elle est séchée sur Mg $SO_4$, filtrée et concentrée à sec. (I) est précipité dans le mélange de solvants ether de pétrole-éther : 410 mg (65 %). Fc 107.110° - $[\alpha]_D^{25}$ - 6,8° (c = 1, méthanol). Anal. calculé pour $C_{15}H_{28}O_5N_2$ (316,40) : C 56,94 - H 8,92 - N 8,85 Trouvé : C 56,68 - H 9,13 - N 8,76

BOC-L-Ala-D-Nle-$NH_2$ (II)

200 mg (0,63 mmoles) de (I) sont ajoutés à une solution, saturée en ammoniac, de méthanol. Après 48 heures à température ambiante, le mélange réactionnel est concentré à sec et (II) précipité dans le mélange de solvants acétate d'éthyle-hexane : 166 mg (87 %).Fc 160-1°- $[\alpha]_D^{25}$ -14,5° (c = 1, méthanol). Anal. calculé pour $C_{14}H_{27}N_4O_3$ (301,39) C 55,79 - H 9.03 - N 13,94 Trouvé : C 55,55 - H 9,17 - N 13,72

.../...

Mur NAc-L-Ala-D-Nle-$NH_2$ (III)

301 mg (1 mmole) de (II) sont traités pendant 30 mn par 3,6 ml d'une solution HCl 1 N dans l'acide acétique. Le mélange réactionnel est concentré à sec, et le résidu obtenu [L-Ala-D-Nle-$NH_2$, HCl] directement utilisé en solution dans 5 ml de DMF.

377 mg (0.8 mmoles) de Mur NAc ((α-Bzl-4,6-O-Bzl). (2) sont dissous dans 5 ml de DMF. A cette solution, sont successivment ajoutés à - 15°, 0,08 ml (0,8 mmoles) de N-méthylmorpholine et 0,10 ml (0,8 mmoles) de chlorocarbonate d'isobutyle, puis, après 3 mn, la solution de L-Ala-D-Nle-$NH_2$, HCl, dans le DMF, aditionnée de 0,11 ml (1 mmole) de N-méthylmorpholine. Après 4 heures à - 15°C, le mélange réactionnel est additionné de 1 ml d'une solution 2,5 M de $KHCO_3$, à 0°C. Après 30 mn, le produit (IIIa) est précipité par addition d'eau. Il est filtré, lavé à l'eau et desséché en présence de $P_2O_5$ sous vide : 384 mg (733 %) Fc 281-290° - $[\alpha]_D^{25}$ = -30,8° (c = 1, acide acétique glacial).

200 mg (0,3 mmole) de (IIIa) sont dissous dans 15 ml d'acide acétique glacial et hydrogénés pendant 10 heures, en présence de 200 mg de palladium 5 % sur charbon. Le mélange réactionnel est filtré, concentré à sec. (III) est obtenu après 2 recristallisations dans le mélange de solvants méthanol-acétone : 70 mg (49.3 %). Fc 220°C- $[\alpha]_D^{25}$ = + 47° (c = 1, acide acétique glacial).

Mur NAc-L-Ala-D-Nle-OMe

D-Nle-OMe, HCl (I)

1,17 g (8,9 mmoles) de D-Nle sont dissous dans 3,5 ml de méthanol absolu. A - 15°C, 0,8 ml (11 mmoles) de chlorure de thionyle sont ajoutés goutte à goutte. Après 2 heures à 40°C, le mélange réactionnel est concentré à sec. (I) est obtenu après précipitation dans méthanol-ether : 1,316 g (81 %). Fc 138.9° - $[\alpha]_D^{25}$ - 23° (c = 1. méthanol) Anal.calculé pour $C_7H_{16}NO_2Cl$ (181,165) : C 46,28 - H 8,37- N 7,71 Trouvé : C 46,19 - H 8,62 - N 7.57 -

BOC-L-Ala-D-Nle-OMe (II)

1,04 g (5,5 mmoles) de BOC-L-Ala sont dissous dans 5 ml de DMF. A cette solution refroidie à - 15°C, sont successivement ajoutés 0,605 ml (5,5 mmoles) de N-methylformamide, 0,715 ml (5,5 mmoles) de chlorocarbonate d'isobutyle, puis, après 3 min, 838 mg (4,6 mmoles) de (I), en solution dans 5 ml de DMF, contenant 0,55 ml de N-methylmorpholine. Après 4 heures `à - 15°C d'une solution $KHCO_3$ 2,5 M sont ajoutés au mélange réactionnel, qui après 30 min, est dilué à l'eau, extrait à l'acétate d'éthyle. La pose organique est successivement lavée à l'eau, avec une solution $KHSO_4$ 5 % à l'eau, avec une solution $NaHCo_3$ M, à l'eau, puis séchée sur Mg $SO_4$, filtrée, concentrée à sec : 1,38 g. (II) est purifié sur colonne de silice à l'aide du mélange de solvants chloroforme-acétate d'éthyle 4-1 (v-v) : 1,31 g (90 %) (Il est obtenu sous forme d'huile). Anal. calculé pour $C_{15}H_{28}N_2O_5$ (316.403) : C 56,94 - H 8.92 - N 8,85 - Trouvé : C 56.99 - H 8,37 - N 8,90.

.../...

<u>Mur NAc-L-Ala-D-Nle-OMe (III)</u>

1,31 g (4,15 mmoles) de (II) sont traités pendant 30 min par 12,45 ml d'une solution d'HCl 1 N dans l'acide acétique glacial. Le mélange réactionnel est concentré à sec et le résidu obtenu (L-Ala-D-Nle-OMe-HCl) est directement utilisé en solution dans 10 ml de DMF.

2,23 g (4,73 mmoles) de MurNAc (α-Bzl-4,6-O-Bzi) (3) sont dissous dans 10 ml de DMF. A cette solution,refroidie à - 15°C, sont successivement ajoutés 0,52 ml (4,73 mmoles) de N-méthylmorpholine, 0,615 ml (4,73 mmoles) de chlorocarbonate d'isobutyle, puis, après 3 min, la solution de l'Ala-D-Nle-OMe, HCl, en solution dans le DMF, contenant 0,473 ml (4,15 mmoles) de N-méthylmorpholine. Après 4 heures à - 15°C, 4,7 ml d'une solution $KHCO_3$ 2,5 M sont ajoutés au mélange réactionnel qui, après 30 min, est dilué à l'eau. (III a) précipite, est lavé à l'eau, séché sous vide, en présence de $P_2O_5$. Il est obtenu après cristallisation à partir du méthanol : 2,71 g (97 %). $[\alpha]_D^{25}$ = + 67.7° (c = 1, acide acétique glacial). Anal. calculé pour $C_{35}H_{47}N_3O_{10}$ (669,78) : C 62,76 - H 7.07 - N 6,27 - Trouvé : C 62,73 - H 7.05 - N 6.18

2,7 g (4,04 mmoles) de (III a) sont dissous dans 100 ml d'acide acétique glacial, et hydrogénés pendant 40 heures en présence de 1 g de palladium 5 % sur charbon. Le mélange réactionnel est filtré, concentré à sec. (III) est purifié sur colonne de silice, à l'aide du mélange de solvants chloroforme-méthanol 6-1 (v-v) puis sur colonne de AG 50 W X 2, diluée à l'eau. Le produit est obtenu sous forme lyophilisé : 1,24 g (62 %). $[\alpha]_D^{25}$ + 35,3° (c = 1, acide acétique glacial) Anal. calculé pour $C_{21}H_{37}N_3O_{10}$ (491.55) : C 51.31 - H 7.59 - V 8,55 - Trouvé : C 51.00 - H 7.94 - N 8,32

Mur NAc-L-Ala-D-Nle-O$\underline{n}$Bu

BOC-L-Ala-D-Nle-OH (I)

607 mg (2,4 mmoles) de BOC-L-Ala-OSu (1) sont ajoutés à une solution, refroidie à 0°C, de 234,3 mg (1,79 mmoles) de D-Nle dans 8 ml de DMF contenant 0,22 ml (2 mmoles) de N-méthylmorpholine. Après 24 heures à température ambiante 0,052 ml (0,4 mmole) de DMAPA (diméthylaminopropylamine) sont ajoutés au mélange réactionnel qui est, après 1 h, concentré à sec. Le résidu obtenu est dissous dans l'acétate d'éthyle, puis lavé successivement par une solution HCl 2M refroidie à 0° et à l'eau jusqu'à pH neutre. La phase organique est séchée sur $Na_2SO_4$ puis concentrée à sec. (I) est précipité dans le mélange de solvants acétate d'éthyle-hexane : 268 mg (50 %). Fc 104-6°C - $[\alpha]_D^{25}$ = - 21° (c = 1, méthanol). Anal. calculé pour $C_{14}H_{26}N_2O_5$ (302,37) C.55,61 - H 8,67 - N 9,26. Trouvé : C 55,3 - H 8,34 - N 8,89.

BOC-L- Ala-D-Nle-O$\underline{n}$Bu (II)

340 mg (1,1 mmoles) de (I) sont dissous dans 4,5 ml de méthanol et 0,5 ml d'eau. Une solution 20 % de $C_sCO_3$ est ajoutée jusqu'à obtenir un pH de 7. Le mélange est concentré à sec, le résidu repris dans 5 ml de DMF, qui est évaporé sous vide (trois fois successivement) pour être finalement dissous dans 3 ml de DMF. La solution est additionnée de 0,135 ml (1,2 mmoles) de bromure de $\underline{n}$ butyle. Après 24 heures, le mélange réactionnel est concentré à sec ; le résidu obtenu est repris dans l'acétate d'éthyle et lavé successivement avec une solution d'acide citrique 10 %, à l'eau, avec une solution $NaHCO_3$M, à l'eau, puis séché sur $MgSO_4$.

La phase organique est filtrée, concentrée à sec (II) est obtenu sous forme d'une huile : 403 mg (100 %)

Mur Nac-L-Ala-D-NLe-On Bu (III)

403 mg (1,12 mmoles)de (II)sont traités pendant 30 min par 3,4 ml d'une solution d'HCl N dans l'acide acétique glacial. Le mélange réactionnel est concentré à sec et le résidu obtenu (L-Ala-D-Nle-On Bu, HCl) directement utilisé, en solution dans 5 ml de DMF.

471 mg (1 mmole) de Mur NAc (α- Bzl-4,6-O-Bzi) β ) sont dissous dans 5 ml de DMF. A cette solution refroidie à - 15°C, sont successivement ajoutés 0,11 ml (1 mmole) de N-méthylmorpholine et 0,13 ml (1 mmole) de chlorocarbonate d'isobutyle, puis après 3 minutes, la solution de L-Ala-D-Nle-On $C_4H_9$, HCl dans le DMF, additionnée de 0,123 ml de N-méthylmorpholine. Après 4 heures à - 15°et une nuit à température ambiante, sous agitation, 2 ml de $NaHCO_3$ sont ajoutés, puis après 30 min, (IIIa) est précipité par addition d'eau, puis lavé à l'eau et desséché en présence de $P_2O_5$ : 690 mg (97 %).Fc 218-221° C -$[\alpha]_D^{25}$ + 90° (c = 1, DMF).

585 mg (0,8 mmoles) de(IIIa)sont dissous dans 15 ml d'acide ascétiqu glacial, et hydrogénés pendant 40 heures, en présence de 585 mg de Pd 5 % sur charbon. Le mélange réactionnel est filtré puis concentré à sec : 491 mg. (III) est purifié sur colonne de silice, à l'aide du mélange de solvants chloroforme-méthanol-acide acétique 60-15-2 (v-v), puis lyophilisé : 383 mg (65 %). $[\alpha]_D^{25}$ = + 35,3° (c = 1, acide acétique glacial).

Anal. calculé pour $C_{24}H_{43}N_3O_{10}$ ; 0,5 $CH_3$ COOH, $1H_2O$(581,64) : C 51,62 - H 8,14 - N 7.22 Trouvé : C 51,56 - H 8,17 - V 7.30 -

Mur NAc-Gly-D-Nle-On-Bu

D-Nle-On Bu, Pts (I)

1,171 g (8,9 mmoles) de D-Nle, 2 g (10,5 mmoles) du monohydrate de l'acide p-toluène sulfonique et 10 ml de n-butanol, en solution dans le benzène sont chauffés à reflux. Après contrôle en chromatographie sur couche mince de gel de silice (chloroforme-méthanol 4-1 v-v), le mélange réactionnel est concentré à sec. (I) est purifié sur colonne de silice, à l'aide du mélange de solvants chloroforme-méthanol 6-1 (v-v), et obtenu sous forme lyophilisé : 2,55 g (80 %). Anal. calculé pour $C_{17}H_{29}NO_5S$ (359,49) : C 56,80 - H 8,13 - N 3,90 - Trouvé : C 55.00 - H 8,08 - N 4,3.

BOC-Gly-D-Nle-On Bu (II)

350 mg (2,2 mmoles) de BOC-Gly sont solubilisés dans 5 ml de DMF. A cette solution refroidie à - 15°C sont ajoutés successivement 0,245 ml (2,2 mmoles) de chlorocarbonate d'isobutyle ,et 0,286 ml (2,2 mmoles) de N-méthylmorpholine, puis, après 3 min, 691 mg (1,92 mmoles) de (I) en solution dans 5 ml de DMF, contenant 0,22 ml (2mmoles) de N-méthylmorpholine. Après 4 h à -15°C, le mélange réactionnel est additionné de 2,2 ml d'une solution à 2,5 M $KHCO_3$.

après 30 min dilué à l'eau et extrait à l'acétate d'éthyle. La phase organique est lavée à l'eau, avec une solution de KH $SO_4$ 5 %, à l'eau, puis après séchage sur Mg $SO_4$ et filtration, concentré à sec. (II) est purifié sur colonne de silice, à l'aide du mélange de solvants chloroforme-méthanol 10 -1 (v-v) et est obtenu sous forme d'huile. Anal. calculé pour $C_{17}H_{32}N_2O_5$ (344,46). C 59,28 - H 9,36 - N 8,13 - Trouvé C 59.03 - H 9,36 - N 7,96

Mur NAc-Gly-D-Nle-On Bu (III)

400 mg (1,16 mmoles) de (II) sont traités par 3,6 ml d'une solution d'HCl N dans l'acide acétique glacial, pendant 30 min. Le mélange réactionnel est concentré à sec et le résidu obtenu (Gly-D-Nle-On Bu, HCl) utilisé directement, en solution dans 5 ml de diméthyl-formamide.

625 mg (1,33 mmoles) du Mur NAc (α-Bzl-4,6-O-Bzi) (3) sont dissous dans 5 ml de DMF. A cette solution refroidie addition de 0,146 ml (1,33 mmoles) de N-méthylmorpholine et 0, 173 ml (1,33 mmoles) de chlorocarbonate d'isobutyle, puis après 3 min, la solution de Gly-D-Nle-On Bu, HCl dans le DMF, contenant 0,133 ml (1,21 mmoles) de N- méthylmorpholine. Après 4 heures à - 15°, 1,3 ml d'une solution 2,5 M $KHCO_3$ sont additionnés au mélange réactionnel qui, après 30 min, est dilué à l'eau. (III a) précipite, est lavé à l'eau et desséché en présence de $P_2O_5$ sous vide. Il est purifié sur colonne de silice, à l'aide du mélange de solvants chloroforme-acétone 18-5 (v-v) et obtenu par cristallisation à partir du méthanol : 572 mg (53 %). Fc 212-3 %- $[\alpha]_D$ + 82,5° (c = 1, acide acétique glacial). Anal. calculé pour $C_{37}H_{51}N_3O_{10}$ (697,84). C 63,68 - H 7,37 - N 6,02 Trouvé : C 63,15 - H 7.22 - N 5,96

572 mg (0.82 mmoles) de (III a) sont dissous dans 50 ml d'acide acétique glacial et hydrogénés en présence de 300 mg de Pd 5 % sur charbon pendant 40 heures. Le mélange réactionnel est filtré, concentré, lyophilisé. (III) est purifié sur colonne de silice, à l'aide du mélange de solvants chloroforme-méthanol-acide acétique 6-1-0,2 (v-v).v sur colonne AG50 WX2 dilué avec le mélange de solvants DMF-eau 1-1 (v-v). Il est obtenu sous forme lyophilisé : 314 mg (72.5 %). $[\alpha]_D^{25}$ + 54,3° (c= 1, acide acétique glacial)

Anal. calculé pour $C_{23}H_{41}N_3O_{10}$ (519,60) : C 53,17 - H 7,95 - N 8,08 -
Trouvé : C 53,13 - H 7.80 - N 7,73.

Mur Nac-L-Val-D-Nle-On Bu

D-Nle-On Bu, pts (I)

1,171 g (8,9 mmoles) de D-Nle, 2 g (10,5 mmoles) du monohydrate de l'acide p-toluène sulfonique et 10 ml de n-butanol, en solution dans le benzène sont chauffés à reflux. Après contrôle en chromatographie sur couche mince de gel de silice (chloroforme-méthanol 4-1 v-v), le mélange réactionnel est concentré à sec. (I) est purifié sur colonne de silice, à l'aide de mélange de solvants chloroforme-méthanol 6-1 (v-v), et obtenu sous forme lyophilisé : 2,55 g (80%). Anal. calculé pour $C_{17}H_{29}NO_5S$ (359,49) : C 56,80 - H 8,13 - N 3,90 - Trouvé : C 55.00 - H 8,08 - N 4,3

BOC-L-Val-D-Nle-On-Bu (II)

478 mg (2,2 mmoles) de BOC-L-Val sont dissous dans 5 ml de DMF. A cette solution refroidie à - 15°C sont successivement ajoutés 0,242 ml (2,2 mmoles) de chlorocarbonate d'isobutyle, puis, et 0,286 ml (2,2 mmoles) de N-méthylmorpholine, puis après 3 min. 691 mg (1,9 mmoles) de (I) en solution dans 5 ml de DMF, contenant 0,22 ml (2 mmoles) de N-méthylmorpholine, après 4 heures à -15°C, 2,2 ml d'un solution de $KHCO_3$ 2,5 M sont ajoutés au mélange réactionnel qui, après 30 min. est dilué à l'eau et extrait à l'acétate d'éthyle. La phase organique est lavée à l'eau, avec une solution $KH\ SO_4$ 5%, à l'eau, puis séchée sur Mg SOu, filtrée et concentrée à sec : 762 mg. (II) est purifié sur colonne de silice ; à l'aide du mélange de solvants chloroforme-acétate d'éthyle 35-1 (v-v).

Il est obtenu sous forme d'huile : 641 mg (86 %). Anal. calculé pour $C_{20}H_{38}N_2O_5$ (386,54) : C 62,15 - H 9.90 - N 7,25 - Trouvé : C 62,19 - H 9.40 - N 7.11

Mur NAc-L-Val-D-Nle-O<u>n</u> Bu (III)

626 mg (1,62 mmoles) de (II) sont traités pendant 30 min par 4, 9 ml d'une solution d'HCl IN dans l'acide acétique glacial. Le mélange réactionnel est concentré à sec et le résidu obtenu (L-Val-D-Nle-On Bu- HCl) utilisé directement, en solution dans 5 ml de DMF.

872 mg (1,85 mmoles) de Mur NAc (α-Bzl-4,6-O-Bzi) (3) sont dissous dans 5 ml de DMF. A cette solution refroidie à - 15° C sont successivement ajoutés 0,204 ml (1,85 mmoles) de N-méthylmorpholine, 0.240 ml (1,85 mmoles) de chlorocarbonate d'isobutyle, puis, après 3 min., la solution de L-Val-D-Nle-On Bu-HCl dans le DMF, contenant 0,185 ml (1,68 mmoles) de N-méthylmorpholine. Après 4 heures à - 15° C, 1,8 ml d'une solution $KHCO_3$ 2,5 M sont ajoutés au mélange réactionnel qui, après 30 min, est dilué à l'eau. (IIIa) précipite, est lavé à l'eau et desseché en présence de $P_2O_5$ sous vide : 904 mg (78 %) Fc 226 - 7°C. $[\alpha]_D^{25}$ = + 67,7° (c = 1. acide acétique glacial). Anal. calculé pour $C_{40}H_{57}N_3O_{10}$ (739,917) : C 64,93 - H 7,76 - N 5,68 - Trouvé : C 64,73 - H 7.78 - N 5,62

904 mg (1,22 mmoles) de (IIIa) sont dissous dans 50 ml d'acide acétique glacial et hydrogénés pendant 40 heures, en présence de 460 mg de palladium 5 % sur charbon. Le mélange réactionnel est filtré, concentré à sec, lyophilisé 723 mg.

(III) est purifié sur colonne de silice, à l'aide du mélange de solvants chloroforme-méthanol-acide acétique 7-1-0,5 (v-v), puis sur colonne de AG 50 W X 2, diluée avec le mélange de solvants DMF - eau = 1-1 (v-v). Il est obtenu sous forme lyophilisée : 558 mg (91%) $[\alpha]_D^{20}$ = + 21° (c = 0.4 acide acétique glacial). Anal. calculé pour $C_{26}H_{47}N_3O_{10}$ (551,68) : C 55,59 - H 8,42 - N 7.48 - Trouvé : C 55,95 - H 8,21 - N 7.53

On peut obtenir de la même façon des dérivés glycopeptidiques dans lesquels X aura une autre signification en partant des acides aminés correspondants BOC-L-X-Nle-Y, dans lesquels X et Y ont les significations sus-indiquées.

Mur NAc-L-Ala-D-Nle-On Hex

BOC-L-Ala-D-Nle-OH (I)

607 mg (2,4 mmoles) de BOC-L-Ala-OSu (1) sont ajoutés à une solution, refroidie à 0°C, de 234,3 mg (1,79 mmoles) de D -Nle dans 8 ml de DMF contenant 0,22 ml (2 mmoles) de N-m éthylmorpholine. Après 24 heures, à température ambiante 0,052 ml (0,4 mmole) de DMAPA sont ajoutés au mélange réactionnel qui est, après une heure, concentré à sec. Le résidu obtenu est dissous dans l'acétate d'éthyle, puis lavé successivement par une solution HCl 2M refroidie à 0° et à l'eau jusqu'à pH neutre. La phase organique est séchée sur $Na_2So_4$ puis concentrée à sec. (I) est précipité dans le mélange de solvants acétate d'éthyle-hexane : 268 mg (50 %)- Fc 104-6°C - $[\alpha]_D^{25}$ = - 21° (c = 1, méthanol). Anal. calculé pour $C_{14}H_{26}N_2O_5$ (302,37) C 55,61 - H 8,67 - N 9,26 - trouvé : C 55,33 - H 8,34 - N 8,89.

BOC-L-Ala-D-Nle-On Hex (II)

268 mg (0,89 mmoles) de (I) sont dissous dans 4,5 ml de méthanol et 0,5 ml d'eau. Une solution 20 % de $C_sCO_3$ est ajoutée jusqu'à obtenir un pH de 7. Le mélange est concentré à sec, le résidu repris dans 5 ml de DMF, qui est évaporé sous vide (trois fois successivement) pour être finalement dissous dans 3 ml de DMF. La solution est additionnée de 0,144 ml (1,02 mmoles) de bromure de n hexyle. Après 24 heures, le mélange réactionnel est concentré à sec, le résidu obtenu est repris dans l'acétate d'éthyle et lavé successivement avec une solution d'acide citrique 10 %, à l'eau avec une solutic $NaHCO_3M$, à l'eau, puis séché sur $MgSO_4$.

La phase organique est filtrée, concentrée à sec (II) est purifié sur colonne de silice, à l'aide du mélange de solvants Hexane-Acétate d'éthyle 3-1 (v-v). Il est obtenu sous forme d'une huile : 290 mg (85 %)

Mur NAc-L-Ala-D-Nle-On Hex (III)

290 mg (0,75 mmoles) de (II) sont traités pendant 30 min par 2,25 ml d'une solution d'HCl N dans l'acide acétique glacial. Le mélange réactionnel est concentré à sec et le résidu obtenu (L-Ala-D-Nle-On Hex, HCl) directement utilisé, en solution dans 5 ml de DMF.

403,5 mg (0,856 mmole) de Mur NAc (α-Bzl-4,6O-Bzi) (3) sont dissous dans 5 ml de DMF. A cette solution refroidie à - 15°C, sont successivement ajoutés 0,094 ml (0,856 mmole) de N-méthylmorpholine et 0,111 ml (0,094 mmole) de chlorocarbonate d'isobutyle, puis après 3 min, la solution de l'Ala-D-Nle-On Hex, HCl dans le DMF, additionnée de 0,086 ml de N-méthylmorpholine. Après 4 heures à - 15° et une nuit à température ambiante, sous agitation, 1 ml de $NaHCO_3$ sont ajoutés, puis après 30 min, (III a) est précipité, par addition d'eau, puis lavé à l'eau et desséché en présence de $P_2O_5$. Il est obtenu par cristallisation à partir du méthanol : 408 mg (73 %). $[\alpha]_D^{25}$ + 68,4° (c = 1, acide acétique glacial) Anal. calculé pour $C_{40}H_{57}N_9O_{10}$ (739,917) : C 64,93 - H 7,76 - N 5,68 - Trouvé : C 64,91 - H 7,61 - N 5,61

540 mg (0,73 mmoles) de (IIIa) sont dissous dans 50 ml d'acide ascétique glacial, et hydrogénés pendant 40 heures, en présence de 250 mg de Pd 5 % sur charbon.

Le mélange réactionnel est filtré, puis concentré à sec. (III) est purifié sur colonne de silice, à l'aide du mélange de solvants chloroforme-méthanol 7-1 (v-v) puis sur colonne d'A6-50 W X 2, diluée à l'eau, puis lyophilisé : 225 mg (55 %). $[\alpha]_D^{27}$ = + 36° (c = 1, acide acétique glacial).

Anal. calculé pour $C_{25}H_{47}N_3O_{10}$ (581,64) : C 55,60 - H 8,43 - N 7.48

Trouvé : C 55,37 - H 8,36 - N 7,28

L'homme du métier appréciera qu'il est possible d'avoir accès aux autres composés conformes à l'invention, par exemple à des composés préférés dans lesquels Y est $NHR_7$, par le choix approprié des réactifs de départ et des groupes protecteurs à mettre en oeuvre, et en adaptant en conséquence les techniques décrites à titre d'exemples dans ce qui précède.

Nor Mur NAc-L-Ala-D-Nle-On Bu

D-Nle-On Bu, Pts (I)

1,171 g (8,9 mmoles) de D-Nle, 2 g (10,5 mmoles) du monohydrate de l'acide p-toluène sulfonique et 10 ml de n-butanol, en solution dans le benzène sont chauffés à reflux. Après contrôle en chronatographie sur couche mince de gel de silice (chloroforme-méthanol 4-1 v-v), le mélange réactionnel est concentré à sec. (I) est purifié sur colonne de silice, à l'aide du mélange de solvants chloroforme-méthanol 6-1 (v-v), et obtenu sous forme lyophilisé : 2,55 g (80 %). Anal. calculé pour $C_{17}H_{29}NO_5S$ (359,49) : C 56,80 - H 8,13 - N 3,90 - Trouvé : C 55.00 - H 8,08 - N 4,3 -

BOC-L-Ala-D-Nle-On Bu (II)

416 mg (2,2 mmoles) de BOC-L-Ala sont dissous dans 5 ml de DMF. A cette solution, refroidie à - 15°C, sont successivement ajoutés 0,242 ml (2,2 mmoles) de chlorocarbonate d'isobutyle, et 0,286 ml (2,2 mmoles) de N-méthylmorpholine, puis après 3 min, 691 mg (1,9 mmoles) de (I) en solution dans 5 ml de DMF, contenant 0,22 ml (1,9 mmoles) de N-méthylmorpholine. Après 4 h à -15°C, 2,2 ml de $KHCO_3$ 2,5 M sont ajoutés au mélange réactionnel qui, après 30 min, est dilué à l'eau et extrait à l'acétate d'éthyle. La phase organique est successivement lavée à l'eau, avec une solution $KHSO_4$ 5 % à l'eau séchée sur $MgSO_4$, filtrée et concentré à sec : 732 mg. (II) est purifiée sur colonne de silices, à l'aide du mélange de solvants chloroforme-méthanol 10-1 (v-v-) et est obtenu sous forme d'huile : 583 mg (85 %).

Anal. calculé pour $C_{18}H_{39}N_2O_5$ (358,48) : C 60,30 - H 9,56 - N 7,81 - Trouvé : C 60,26 - H 9.45 N 7,61.

Nor Mur NAc-L-Ala-D-Nle-On Bu (III)

586 mg (1,58 mmoles) de (II) sont traités, pendant 30 min par 4,8 ml d'une solution 1 N d'HCl dans l'acide acétique glacial. Le mélange réactionnel est concentré à sec et le résidu obtenu (L-Ala-D-Nle- On Bu, HCl) utilisé directement en solution dans 5 ml de DMF.

530 mg (1,15 mmoles) de Nor Mur NAc (β-Bzl-4,6-Bzi) (4) sont dissous dans 10 ml de DMF. A cette solution, refroidie à - 15°C, sont successivement ajoutés 0,125 ml (1,15 mmoles) de N-méthylmorpholine, 0,150 ml (1,15 mmoles) de chlorocarbonate d'isobutyle, puis après 3 minutes, la solution de L-Ala-D-Nle-On Bu, HCl dans la DMF, contenant de 0,17 ml (1,6 mmoles) de N-méthylmorpholine. Après 4 heures à - 15° C, 1,2 ml d'une solution de $KHCO_3$ 2,5 M est ajouté au mélange réactionnel qui, après 30 min, est dilué à l'eau. (III a) précipite, est lavé à l'eau, séché en présence de $P_2O_5$ sous vide. (III a) est purifié sur colonne de silice, à l'aide du mélange de solvants chloroforme-acétone 18-5 (v-v) et est obtenu après cristallisation à partir du méthanol : 553 mg (75 %). Fc 233-4°C - $[\alpha]_D^{25}$ - 57,4° (c = 1, acide acétique glacial). Anal. calculé pour $C_{37}H_{51}N_3O_{10}$ (697.836) : C 63,68 - H 7,37 - N 6,02 - Trouvé : C 63,36 - H 7,28 - N 5,93 -

553 mg (0,79 mmoles) de (III a) sont dissous dans 40 ml d'acide acétique glacial et hydrogéné pendant 40 heures en présence de 270 mg de palladium 5 % sur charbon. Le mélange réactionnel est filtré et concentré à sec.

.../...

(III) est purifié sur colonne de silice, à l'aide du mélange de solvants chloroforme-méthanol 8-1, puis sur colonne de AG SOW X 2, diluée à l'eau. (III) est obtenu sous forme lyophilisée : 262 mg (64 %). $[\alpha]_D^{21}$ + 4,6° (c = 0.5, acide acétique glacial). Anal. calculé pour $C_{23}H_{41}N_3O_{10}$ (519.602). C 53,47 - H 7.95 - N 8,08 - Trouvé : C 53,46 - H 7,89 - N 7.72.

Ont encore été obtenus les dérivés suivants en mettant en oeuvre des procédés tels que décrits ci-dessus :

1) 6-O-ε-aminocaproyl-MurNAc-L-Ala-D-Nle-OnBu ;
2) 6-amino-6-deoxy-MurNAc-L-Ala-D-Nle-OnBu
3) 6-dipalmitoyl-glyceroyl-6-deoxy-MurNAc-L-Ala-D-Nle-OnBu ;
4) 6-dipalmitoyl-glyceroyl-ε-aminocaproyl-MurNAc-L-Ala-D-Nle-OnBu ;

les caractéristiques analytiques de ce produit étant les suivants :

$[\alpha]_{25}^{D}$ = + 15,5° (C : 0,3 ; $CH_3COOH$, 2H d'équilibre)
Formule brute = $C_{65}H_{118}N_4O_{16}$ + 0,9 $CH_3COOH$
Analyse quantitative = en %.

Calculé :

C = 63,69
H = 9,69
N = 4,45

Trouvé :

C = 63,40
H = 9,69
N = 4,70

<u>Mur-NAc-L-Ala-D-NLe-$C_4H_9$</u>

<u>N-trityl-D-norleucine, sel de diéthylammonium</u> (I)

$C_{29}H_{38}N_2O_2$ : 446,6.

---

A 2,6 g de D-Nle (20 mmoles) en solution dans 30 ml d'un mélange isopropanol-eau (1/1) en présence de 6,3 ml de diéthylamine (90 mmoles), addition par portions en 7 heures de 7,25 g de chlorure de trityle (26 mmoles), fraîchement recristallisé dans l'hexane. Après 1 heure d'incubation à température ambiante et contrôle chromatographique dans $CHCl_3$-$CH_3$ OH : 15/1, la réaction est arrêtée par filtration du précipité formé.

m : 2,2 g (25 %).

<u>N-trityl-D-norleucine-thiopyridinyl-ester</u> (II)

$C_{25}H_{27}NO_2$ : 373,5.

2,21 g de (I) (5 mmoles) en suspension dans 20 ml d'un mélange AcOEt-$H_2O$ (1/1) sont traités par 1 volume d'acide citrique à 10 %. Après décantation, lavages à l'eau saturée en NaCl jusqu'à pH neutre, séchage sur $Na_2SO_4$, concentration.

m : 1,67 g (huile) ; 90 %.

Après solubilisation dans 15 ml d'acétate d'éthyle (AcOEt) <u>sec</u>, addition d'un équivalent de mercaptopyridine (4,47 mmoles). A la solution agitée en incubation à -20°C, addition au goutte à goutte d'un équivalent de dicyclohexyl-carbodiimide en solution dans l'AcOEt sec (25 ml). Après une nuit d'incubation à température ambiante, le précipité de dicyclohexylurée est filtré. Le filtrat et les lavages, sont lavés successivement par $NaHCO_3$ saturé et l'eau saturée en NaCl. Séchage sur $Na_2SO_4$, concentration. Purification sur colonne de silice 60 G par élution à l'aide du mélange Hexane-Acétate d'éthyle (4/1).

m : 1,37 g (poudre amorphe) ; 74 %.

$[\alpha]_D^{25}$ : 102° (c : 0,5, THF).

Analyse élémentaire :

$C_{25}H_{27}NO_2$ (373,5).

Calculé % : C 77,22 ; H 6,48 ; N 6,00.

Trouvé % : C 77,27 ; H 6,50 ; N 5,91.

6(R)-N-(trityl)-amino-décanone 5 (III).

$C_{29}H_{35}NO$ : 413,6.

---

A 1,35 g de (II) (2,9 mmoles), en solution dans 25 ml de THF sec incubés au bain-marie à 0°C et agités en conditions anhydres sous atmosphère d'azote, addition au goutte à goutte de 2,5 ml d'une solution de bromure de butylmagnésium dans le THF sec (1,2 mmole/ml). La réaction est arrêtée, après une heure et demie d'incubation, par addition de 10 ml de $NH_4Cl$ saturé, et extension à l'éther. Lavages successifs par NaOH N et NaCl saturé ; séchage sur $Na_2SO_4$ ; concentration. Purification sur colonne de silice 60 G à l'aide du mélange Hexane-Acétate d'éthyle (12/1).

m : 720 mg (huile) ; 60 %.

6(R)-amino-décanone-5, oxalate (IV)

$C_{10}H_{21}NO$, 1 HOOC-COOH : 261,3.

---

690 mg de (III) (1,7 mmoles) sont incubés une heure à température ambiante en présence d'une solution de 225 mg d'acide oxalique dans 15 ml d'un mélange acide acétique-eau (2/1), soit 1,05 équivalent. Après extension par un volume d'eau, le précipité de triphenylcarbinol est filtré et lavé à l'eau. Le filtrat et lavages sont concentrés, (IV) est obtenu sous forme de lyophilisat à partir de sa solution dans l'eau.

m : 450 mg.

N-acétylmuramyl-L-alanyl-6(R)-amino-décanone-5 (V).

$C_{24}H_{43}N_3O_9$ : 517,62

---

ou encore MUR-NAC-L-ALA-D-NLe-$C_4H_9$ (V)

570 mg de Mur-Nac-L-Ala (1,5 mmole) en solution dans 15 ml de DMF en présence d'un équivalent de N-méthylmorphine sont incubés à -15°C pendant 30 minutes en présence d'un équivalent de chloroformiate d'isobutyle. Addition de 350 mg de (IV), soit 1,25 mmole, en solution dans 15 ml de DMF sec en présence d'un équivalent de N-méthylmorpholine et incubation pendant 4 heures à -15°C. Concentration au minimum, extension à l'eau, lyophilisation. Purification sur colonne de silice 60 G à l'aide d'un mélange $CHCl_3$-$CH_3OH$-$CH_3COOH$ : 8/1/0,1. Concentration. Percolation sur colonne de résine AG 50 $WX_2$ (Biorad) dans l'eau. (IV) est obtenu sous forme de lyophilisat à partir de sa solution dans l'eau.

m : 422 mg (poudre amorphe) ; 65 %.

$[\alpha]_D^{25}$ : + 33,5° (c = 0,5, $H_2O$, 2 H d'équilibre).

Analyse élémentaire :

$C_{24}H_{43}N_3O_9$ + 0,7 $H_2O$ (530,24).

calculé % : C 54,36 ; H 8,44 ; N 7,92.

Trouvé % : C 54,39 ; H 8,30 ; N 7,90.

A titre d'exemple de conjugués préférés susceptibles d'être fabriqués avec des composés préférés de l'invention, on mentionnera ceux réalisés à partir des composés du type 6-O-succinyl-MurNac-L-Ala-D-Nle-$NH_2$, ou 6-O-succinyl-MurNac-L-Ala-D-Nle-OnBu, ou encore les dérivés 6-O-ε-amino-caproyle correspondants, d'une part, et d'haptènes dont certains sont indiqués ci-après à titre d'exemples préférés : le facteur de libération de la lutéotropine, connue sous la désignation LH-RH, des fragments peptidiques, ou des peptides synthétiques doués de propriétés analogues, par exemple des décapeptides du type

Glu-His-Trp-Ser-Tyr-Gly-Leu-Arg-Pro-Gly-$NH_2$

ou l'acide libre correspondant

pGlu-His-Trp-Ser-Tyr-Gly-Leu-Arg-Pro-Gly.

- des peptides C-terminaux de la gonadotropine chorionique humaine (hCG),
- des peptides C-terminaux de la sous-unité β de la hCG, notamment un triacontapeptide contenant les résidus aminoacyles correspondant à ceux qui se trouvent dans l'hCG-β ou de dérivés synthétiques terminaux du genre de ceux décrits par S. MATSUURA et al (publication déjà mentionnée plus haut), des fragments de FSH, etc, ou des peptides consistant essentiellement en les séquences 109-145 et 111-145 des sous-unités C-terminales de hCG-β décrites par Arthur C.J. LEE et al dans "Molecular Immunology", vol. 17, p. 749-756.

La modification des hormones qui précèdent par couplage avec des glycopeptides tels que mentionnés ci-dessus conduit à des principes immunogènes susceptibles d'induire in vivo la production d'anticorps contre les hormones naturelles en question, immunogénicité d'autant plus remarquable que les haptènes modifiés immunogènes ainsi obtenus ont des poids moléculaires extrêmement faibles.

On mentionnera encore, à titre d'exemples de conjugués mettant en jeu le couplage entre un glycopeptide

conforme à l'invention et d'haptènes, ceux qui mettent en jeu différents peptides synthétiques ayant des séquences de résidus aminoacyles communs avec les principaux peptides constitutifs de l'antigène HBsAg, notamment ceux dont les aminoacyles d'extrémité correspondent à ceux qui, dans la séquence de HBsAg occupent les positions suivantes : 48-81 ; 2-16 ; 22 - 35 ; 95 - 100 ; 117 - 137 ; 122 - 137 ; 117 - 135 (selon la structure donnée par PASEK et al et reproduite dans l'article de LERNER).

On citera en outre des peptides synthétiques du genre de ceux décrits par E.H. BEACHEY et al qui sont susceptibles, lorsque couplés avec un muramylpeptide, de former des anticorps actifs contre la protéine M de la surface de Streptococcus pyogenes, cette protéine désignée plus loin dans l'exemple II, sous l'abréviation "M24". On peut également se référer aux séquences peptidiques définies dans le brevet américain n° 4 284 537, plus particulièrement celles définies sous les abréviations $CB_6$ et $CB_7$.

Des haptènes peptidiques particulièrement préférés mis en oeuvre dans des conjugués conformes à l'invention sont un ou plusieurs de ceux définis ci-après :

1°) SODP : octodécapeptide contenant une séquence de la "boucle diphtérique" et de formule :

$H_2N$-Ala-Ala-Cys-Ala-Gly-Asn-Arg-Val-Arg-
Arg-Ser-Val-Gly-Ser-Ser-Leu-Lys-Cys.

2°) SCB7 : peptide contenant un épitope caractéristique du streptococcus type M24 contenant la séquence :

Asn-Phe-Ser-Thr-Ala-Asp-Ser-Ala-Lys-
Ile-Lys-Thr-Leu-Glu-AlaGlu-Lys-Ala-Ala-
Leu-Ala-Ala-Arg-Lys-Ala-Asp-Leu-Glu-Lys-
Ala-Leu-Glu-Gly-Ala

3°) HBs (peptide 99-121) : peptide comprenant un site antigénique de l'enveloppe du virus contenant la séquence :

Asp-Tyr-Gln-Gly-Met-Leu-Pro-Val-Cys-Pro-Leu-Ile-
100 110
Pro-Gly-Ser-Ser-Thr-Thr-Ser-Thr-Gly-Pro-Cys.
120

4°) DDP Mal : peptide portant un épitope caractéristique d'un antigène protecteur contre *Plasmodium Knowlesi*, agent de la malaria du singe, décrit par GODSON G.N. et Coll. (1983), Nature, 305, 29-33, constitué par la séquence :

Tyr-Gln-Ala-Gln-Gly-Asp-Gly-Ala-Asn-Ala-
Gly-Gln-Pro-Gln-Ala-Gln-Gly-Asp-Gly-Ala-Asn-
Ala-Gly-Gln-Pro-Cys

Ces exemples n'ont bien entendu aucun caractère limitatif. En outre, les haptènes utilisables ne sauraient être limités à des peptides. Il s'agit par exemple aussi d'haptènes saccharidiques ou osidiques. On mentionnera par exemple les groupes sanguins A et B, le polysaccharide C du Streptococcus C, l'acide cellobiuronique (Pneumo type III), tel que décrit par W.F. GOEBEL, "J. Exp. Medicine", 1939, p. 353-363, ou l'acide para-aminophényl-cellobiuronique.

Pour mémoire, on rappelle certaines des techniques préférées pour réaliser la susdite conjugaison.

Conformément à une première technique, on peut avoir recours à une réaction de couplage par l'intermédiaire de glutaraldéhyde, dans la mesure où tant l'haptène que le dérivé de glycopeptide considéré comporte des fonctions amine.

Avantageusement, on peut avoir recours à d'autres formes de couplage pouvant mettre en jeu des positions déterminées sur la partie glycopeptide. En particulier, ces

liaisons pourront intervenir au niveau soit de la position en $C_6$, soit de celle en $C_1$ du cycle saccharidique, soit encore à l'extrémité C-terminale Y de la chaîne peptidique.

En ce qui concerne les substitutions impliquant le groupement $R_6$, on pourra avoir recours aux méthodes de couplage couramment utilisées en synthèse peptidique, dans la mesure où l'on pourra réaliser une liaison peptidique entre des fonctions appropriées portées, d'une part, par le glycopeptide et, d'autre part, par l'haptène, l'un portant par exemple une fonction amine et l'autre une fonction carboxyle, hydroxyle ou sulfhydrile ou vice-versa. Il va de soi qu'il conviendra alors de faire porter au groupe $R_6$ la fonction terminale appropriée, pour réaliser la fixation au niveau du carbone en 6 sur le groupe saccharidique selon des techniques bien connues du spécialiste.

Bien entendu, on peut avoir recours à tout autre type de liaison, mettant en jeu les combinaisons pouvant intervenir entre une fonction sulfhydrile portée par l'un des partenaires que constituent l'haptène et le muramyl-peptide et un groupe maléimide porté par l'autre réactif, par exemple en faisant usage de la technique décrite par T. KITAGAWA et T. AIKAGAWA dans "J. Biochem." 79 (1976), 233.

La conjugaison peut se faire en utilisant les modes classiques de diazotisation ou de réaction mettant en jeu un isothiocyanate, notamment lorsque le muramyl-peptide porte une fonction aromatique aminée (notamment au niveau du groupe $R_1$) et lorsque l'haptène porte une fonction amine susceptible d'intervenir dans cette réaction. De telles techniques de réalisation sont décrites par exemple par B.F. ERLANGER dans "Pharmacol. Rev.", 25 (1973), 271.

Le couplage peut également être réalisé par la réduction d'une base de Schiff réalisée entre une fonction aldéhyde portée par l'un des réactifs et une fonction amine portée par l'autre (G.R. GRAY, "Arch. Biochem. Biophys. 163

(1974), 426).

Toutes ces réactions sont en soi bien connues et le spécialiste appréciera que de nombreuses variantes peuvent être aménagées pour aboutir aux mêmes résultats. En ce qui concerne d'ailleurs plus particulièrement les groupes Z et $R_1$, on remarquera aussi que, entre le glycopeptide et l'haptène, les conjugaisons peuvent s'effectuer au moyen d'un pontage, l'agent de pontage consistant par exemple en un réactif bifonctionnel. En l'occurrence, le groupe de pontage entre le glycopeptide et l'haptène dans le conjugué final de préférence n'excèdera pas la longueur d'un enchaînement correspondant à celle d'une chaîne hydrocarbonée p-décylique.

De tels agents de pontage sont par exemple mentionnés dans le brevet N° 78 16792 déposé le 5 Juin 1978.

Dans ce qui précède, il a surtout été évoqué le cas du couplage entre un glycopeptide et un haptène de nature peptidique. Lorsque l'haptène est constitué par un oligosaccharide, les mêmes types de réactions peuvent être mis en oeuvre, dès lors que l'haptène possède une fonction carboxylique libre ou une fonction amine, ou encore une fonction alcool pouvant alors former des liaisons amide ou ester avec des fonctions adéquates portées par le muramylpeptide.

En variante, on pourra également avoir recours à une fixation du glycopeptide sur la position en $C_1$ de l'oligosaccharide, possédant un sucre terminal réducteur, soit que la fonction pseudoaldéhyde soit oxydée en une fonction carboxylique (G. ASHWELL, "Methods in Enzymol." 28 (1972), 219), soit qu'elle soit utilisée en tant que telle (R. GRAY déjà cité). Dans le premier cas, on établit une liaison peptidique ou ester ; dans le second cas, une liaison alcoylamine.

La fixation de l'haptène sur le glycopeptide peut

intervenir en plusieurs points de celui-ci, l'inverse étant également vrai, de sorte que l'invention s'étend à des oligomères de glycopeptide et de l'haptène, dans la mesure où les deux constituants présentent les fonctions chimiques appropriées. De préférence, le poids moléculaire de tels conjugués ne dépasse pas 5000.

D'une façon générale, le constituant haptène et le constituant glycopeptide seront avantageusement dans un rapport pondéral compris entre 1,5 et 10, avantageusement entre 2 et 3.

L'invention concerne d'ailleurs également des oligomères, des glycopeptides selon l'invention hydrosolubles, contenant notamment de 2 à 10 motifs glycopeptide monomères. Les techniques décrites dans le brevet français N° 78 16792 déposé le 5 Juin 1978 sont directement transposables, pour ce qui est de leur fabrication.

Il va de soi que l'on aura souvent à envisager dans toutes ces réactions l'utilisation de groupes de protection pour les groupes réactifs ne devant pas intervenir dans la réaction de couplage. Ces techniques sont bien connues de l'homme du métier pour qu'il ne soit plus nécessaire d'insister à leur sujet.

Il est d'ailleurs à remarquer que les produits ainsi protégés sont à considérer comme faisant partie de l'invention et comme n'échappant pas à la portée des revendications qui suivent, même si celles-ci ne définissent pas spécifiquement lesdits groupes de protection. Il en va de même encore des sels d'acide ou de base dans lesquels les glycopeptides peuvent entrer, selon la nature des groupes fonctionnels dont ils sont porteurs. Il s'agit plus particulièrement encore des sels physiologiquement acceptables de ces glycopeptides, conjugués ou oligomères.

Propriétés biologiques des glycopeptides selon l'invention

1 - Propriétés immuno-adjuvantes

L'activité immunoadjuvante a été étudiée en mettant en jeu la réponse humorale de la souris swiss femelle à la sérumalbumine bovine.

Des groupes de huit souris âgées de 2 mois reçoivent par voie sous-cutanée 0,5 mg de sérumalbumine bovine avec ou sans les substances à tester, à raison de 100 µg par souris. Les administrations sont faites en solution aqueuse.

Au jour 28, on effectue des prélèvements du sérum sanguin sur les souris traitées et la réponse primaire en anticorps est déterminée par hémagglutination passive en utilisant des hématies de moutons traitées à la formaline et recouvertes de l'antigène étudié selon la méthode décrite par A. A. HIRATA et M.W. BRANDISSE (J.immunol.100, 641 - 648, 1968). Au jour 30 les souris reçoivent une injection de rappel avec les antigènes seuls (100 µg par souris) et au jour 36 on mesure la réponse secondaire induite par la même technique de mesure .

Les résultats de ces essais figurent dans le tableau I qui suit. Ce tableau contient également les résultats mesurés dans les mêmes conditions chez les animaux témoins et chez des animaux qui ont été traités de la même manière avec le MDP à titre de substance de comparaison.

Il est à remarquer que dans le test expérimental utilisé, le composé (1) n'a pas témoigné d'une activité spécifique, au moins à la dose utilisée. En tout état de cause, des composés de ce type font partie de l'invention en ce qu'ils constituent des produits intermédiaires utiles pour la fabrication de produits actifs, par exemple par substitution d'un groupe ester au groupe carboxyle du radical <u>nor</u> Leucine.

TABLEAU I

| Adjuvants (Glycopeptides) | Réponse primaire Jour 28 | Réponse secondaire Jour 36 |
|---|---|---|
| Témoins | < 1,64 | 5,09 ± 2 |
| MurNAc-L-Ala-D-isoGl$_2$ (MDP) | 3,97 | 8,48 ± 1,7 |
| (1) MurNAc-L-Ala-D-Nle-$NH_2$ | - | - |
| (2) MurNAc-L-Ala-D-Nle-On $C_4H_9$ | 1 | 7 |
| (3) MurNAc-Gly-D-Nle-On $C_4H_9$ | 2,64 | 7,83 ± 1,87 |
| (4) MurNAc-L-Val-D-Nle-On $C_4H_9$ | 1,64 | 6,46 ± 1,67 |
| (5) nor MurNAc-L-Ala-D-Nle-On $C_4H_9$ | 1,64 | 7,99 ± 1,93 |
| (6) MurNAc-L-Ala-D-Nle-$OCH_3$ | 1,64 | 7,58 ± 3 |
| (7) MurNAc-L-Ala-D-Nle-On $C_6H_{13}$ | < 1,64 | 6,39 ± 3,1. |

L'examen de ce tableau fait apparaître une activité immunoadjuvante significative des glycopeptides selon l'invention. Toutes les substances présentent une activité qui se rapproche sensiblement (en particulier en ce qui concerne les substances 2) 5) et 6) de celle du MDP. Cependant, contrairement au MDP, des doses répétées de ces subtances n'induisent pas d'activités immunosuppressives. En effet, le MDP comme beaucoup d'immunostimulants, alors qu'il est adjuvant en injection unique à petites doses en même temps que l'antigène, peut inhiber la réponse immunitaire lorsqu'il est administré à doses répétées ou à très fortes doses avant l'antigène.

Dans l'expérience qui figure au tableau Ibis et qui a été répétée 3 fois, le MDP et les autres substances ont été injectés à des souris à une dose 10 ou 20 fois plus élevée que d'habitude (1 mg) 2 jours avant l'injection de $10^8$ globules rouges de mouton. Les splénocytes ont été pélevés et mis en culture 4 jours après l'injection de globules rouges de mouton et le nombre de plages hémolytiques dosées selon la méthode de Cunningham. Alors que ce traitement inhibe totalement la production d'anticorps dans le cas du MDP, du murabutide et des autres dérivés adjuvants précédemment décrits, il y a moins ou même pas du tout d'inhibition pour certaines des autres substances bien que ces dernières aient un pouvoir adjuvant similaire à celui du MDP.

Ces résultats sont également à prendre en considération à la lumière de l'absence pratiquement totale de pyrogénicité des substances selon l'invention, lesquelles présentent par conséquent un index thérapeutique particulièrement élevé.

2- <u>Comportement des substances dans les essais de mesure de l'effet pyrogène</u>.

Les essais sont réalisés sur le lapin selon le protocole de la Pharmacopée Européenne. Les produits à l'étude on été administrés à des lapins à raison de 3 mg/kg. Les élévations de température observées chez chacun d'entre eux résultent du tableau 2 qui suit :

Des résultats sensiblement équivalents ont été obtenus à des doses de 10 mg/kg chez le lapin.

TABLEAU I BIS

| Adjuvants (Glycopeptides) | Pourcentage d'inhibition | |
|---|---|---|
| MurNAc-L-Ala-D-isoGl$_2$ (MDP) | 84,2 | $p < 0,05$ |
| (1) MurNAc-L-Ala-D-Nle-$NH_2$ | NT | |
| (2) MurNAc-L-Ala-D-Nle-On $C_4H_9$ | 3 | NS |
| (3) MurNAc-Gly-D-Nle-On $C_4H_9$ | 37,7 | NS |
| (4) MurNAc-L-Val-D-Nle-On $C_4H_9$ | 10,3 | NS |
| (5)Nor MurNAc-L-Ala-D-Nle-On $C_4H_9$ | 25 | NS |
| (6) MurNAc-L-Ala-D-Nle-$OCH_3$ | 12,3 | NS |
| (7) MurNAc-L-Ala-D-Nle-On $C_6H_{13}$ | 10,9 | NS |

TABLEAU II

| GLYCOPEPTIDES | Doses : | △ T, °C |
|---|---|---|
| Pc 75094 MurNAc-L-Ala-D-Nle-$NH_2$ | NT | NT |
| MurNAc-L-Ala-D-Nle-On $C_4H_9$ | 3 mg/kg | 0,1-0,2-0,0- |
| MurNac-Gly-D-Nle-On-$C_4H_9$ | " | 0,1-0,0-0,1- |
| MurNAc-L-Val-D-Nle-On $C_4H_9$ | " | 0,1-0,4- |
| nor MurNAc-L-Ala-D-Nle-On $C_4H_9$ | " | 0,2-0,1-0,1- |
| MurNAc-L-Ala-D-Nle-$OCH_3$ | " | 0,2-0,1-0,0- |
| MurNAc-L-Ala-D-Nle-On $C_6H_{13}$ | " | 0,2-0,1-0,1- |

NT = non testé

Il résulte de l'examen de ces résultats que les substances selon l'invention sont pratiquement dépourvues de pyrogénicité. En effet, il est communément admis que les substances commencent à induire une activité pyrogène si les élévations de température mesurées dans les conditions qui précèdent dépassent 0,6 °C.

Les résultats obtenus avec les substances selon l'invention sont d'autant plus intéressantes que leurs métabolites sont à la fois inactifs et totalement dépourvus de pyrogénicité..

L'invention concerne également des compositions pharmaceutiques dans lesquelles les glycopeptides (modifiés ou non) sont associés à un véhicule physiologiquement acceptable.

Des compositions pharmaceutiques avantageuses sont constituées par des solutions injectables contenant une dose efficace d'au moins un composé selon l'invention. De préférence, ces solutions sont réalisées dans une phase aqueuse stérilisée isotonique, de préférence saline ou glucosée. L'invention concerne également des compositions dans lesquelles les glycopeptides sont encapsulés dans des liposomes aptes à former des suspensions injectables.

L'invention concerne plus particulièrement de telles solutions ou suspensions qui sont aptes à être administrées par injections intradermiques, intramusculaires ou sous-cutanées, intraveineuses ou encore par scarifications.

L'invention concerne aussi des compositions pharmaceutiques, administrables par d'autres voies, notamment par voie orale ou rectale, ou encore sous des formes destinées à venir en contact avec des muqueuses, notamment les muqueuses oculaires, nasales, pulmonaires ou vaginales.

En conséquence, elle concerne des compositions pharmaceutiques dans lesquelles l'un au moins des composés selon l'invention se trouve associé à des excipients pharmaceutiquement acceptables, solides ou liquides, adaptés à la constitution de formes d'administration orales oculaires ou nasales, ou à des excipients adaptés à la

constitution de formes d'administration rectale. Elle concerne aussi des compositions destinées à la voie pulmonaire, notamment des solutions préparées en vue de l'administration au moyen d'un appareil d'aérosols classique .

A titre d'exemples de doses susceptibles d'être administrées, pour stimuler les réponses immunitaires de l'hôte contre des antigènes déterminés, on mentionnera des doses, en microgrammes par kg de corps, par exemple de 10 à 1000 microgrammes/kg lorsque l' administration est effectuée par voie parentérale, ou de 0,2 à 20 mg/kg par voie orale. L'utilisation des glycopeptides selon l'invention peut se faire en association avec tout agent immunogène dont on veut renforcer la capacité d'induction in vivo d'une réponse immunitaire. L'utilisation des glycopeptides selon l'invention est particulièrement recommandée lorsque l'agent immunogène est faible ou utilisé à très faible dose ou encore lorsque son caractère immunogène a été amoindri, par exemple au cours des modifications ou purifications antérieures auxquelles il a pu être soumis.

L'agent immunogène peut être administré, soit simultanément, soit selon un programme d'administrations décalées vis-à-vis de celle de l'agent immunoadjuvant selon l'invention.

Des compositions vaccinales préférées selon l'invention contiennent en outre un véhicule, tel que la polyvinyl-pyrrolidone, facilitant l'administration du vaccin. A la place de la polyvinyl-pyrrolidone, on peut utiliser tout autre type d'adjuvant au sens classique que l'on donnait autrefois à cette expression, c'est-à-dire d'une substance permettant l'absorption plus aisée d'un médicament ou facilitant son action dans l'organisme. A titre d'exemples, d'autres adjuvants de ce dernier type, on mentionnera encore la carboxyméthylcellulose, les hydroxydes et phosphates d'aluminium ou tous autres adjuvants de ce type, bien connus de l'homme de l'art.

Les compositions pharmaceutiques selon l'invention

sont donc essentiellement destinées à induire chez l'hôte une réaction immunitaire, lui permettant de produire des anticorps ou une immunité à médiation cellulaire à l'égard d'un antigène ou d'un haptène (conjugué ou non au glycopeptide). Il est rappelé que l'expression haptène s'étend (dans le cas de la conjugaison) à des principes naturels, par exemple à un peptide naturel, tel qu'une hormone, dont on veut pallier les effets. Ces compositions pharmaceutiques sont donc particulièrement utiles en thérapeutique humaine ou vétérinaire, chaque fois qu'est recherchée une modification de l'évolution ou du comportement de l'organisme humain ou animal, par exemple au niveau de la fécondité de l'espèce ou de l'orientation dirigée de son métabolisme vers la suppression de certaines hormones. Dans des cas préférés de ce dernier type, l'haptène entrant dans le conjugué mis en oeuvre est constitué par l'hormone elle-même ou un fragment de celle-ci.

Dans le domaine vétérinaire, on citera en outre la production accrue de certains constituants, au détriment d'autres constituants. On citera par exemple les applications des vaccins vétérinaires administrés dans le but d'accroître chez l'animal la production de viande par castration immunologique.

L'invention a en particulier pour objet des compositions vétérinaires contenant des doses efficaces de conjugués d'haptène, dans lesquels l'haptène lui-même est constitué soit par l'hormone elle-même, soit par une séquence peptidique ayant une partie commune avec l'hormone, l'hormone appartenant à la classe des hormones peptidiques ou glycopeptidiques. Une hormone particulièrement intéressante à cet égard est constituée par le facteur de libération de l'hormone lutéostimulante LH-RH.

Des doses unitaires efficaces de ces conjugués sont, à titre d'exemples, comprises entre environ 0,1 et 2 mg/kg de corps, lorsqu'ils sont administrés par voie

parentérale, et entre environ 0,2 et 20 mg/kg lorsqu'ils sont administrés par voie orale.

L'invention concerne également encore des réactifs biologiques essentiellement formés à partir de ces conjugués haptène-glycopeptides. En particulier, la partie hapténique peut être constituée par un principe actif de médicament ou de drogue (par exemple la morphine), le réactif biologique en question étant alors utilisé pour fabriquer des anticorps de pyrogénicité réduite, notamment d'anticorps monovalents ou monoclonaux. Ces anticorps sont alors à leur tour utiles pour la constitution de réactifs permettant par exemple l'étude de l'action de médicaments en cause sur l'hôte vivant. En particulier, ces anticorps permettront la détection de récepteurs cellulaires, ou encore l'étude du métabolisme et du mode d'action des médicaments en cause.

Des composés lipophiles parmi les composés selon l'invention ont également une activité anti-infectieuse, anti-virale ou anti-tumorale.

L'invention ne se limite évidemment pas aux modes de réalisation décrits ci-dessus à titre d'exemples et l'homme de l'art peut y apporter des modifications sans pour autant sortir du cadre des revendications ci-après.

Par exemple, il va de soi que ne sortiraient pas du cadre des revendications tous glycopeptides modifiés qui posséderaient des propriétés immunoadjuvantes semblables et qui présenteraient les mêmes éléments caractéristiques de structure, mais qui ne différeraient de ceux qui ont été plus particulièrement envisagés que par des substitutions locales de groupes non spécifiquement envisagés dans les définitions qui en ont été données ou qui déborderaient celles-ci. Par exemple seraient des équivalents des muramyl-peptides modifiés de l'invention, ceux qui comporteraient des groupes de substitution sur la position 4 du groupe

saccharidique, par exemple des groupes acyle comprenant de 1 à 4 atomes de carbone. Seraient encore des équivalents des composés revendiqués, ceux dans lesquels le résidu amino-acyle séparant le groupe NAcMur et le groupe norLeucine serait lui-même modifié, par exemple N-substitué par un groupe alcoyle inférieur, plus particulièrement méthyle.

En conclusion, il importe de souligner que ces diverses substitutions ne sauraient faire sortir les composés ainsi modifiés du cadre de l'invention, dès lors qu'elles ne modifieraient pas les propriétés essentielles, notamment immunoadjuvantes, des glycopeptides ainsi obtenus.

Il va sans dire que sont également des équivalents des composés revendiqués tous les sels et esters biologiquement acceptables dans lesquels composés selon l'invention sont susceptibles d'entrer, dès lors qu'ils possèdent des groupes fonctionnels les rendant capables de les former par réaction avec un réactif complémentaire approprié, tel qu'une amine ou un acide physiologiquement acceptable.

REVENDICATIONS

1. Composé choisi parmi les glycopeptides du type 2-(2-acétamido-2-désoxy-3-OD--glucopyranosyl)alcanoyl-amino acyl-norLeucine et les dérivés desdits glycopeptides ayant des propriétés immuno-adjuvantes.

2. Composé selon la revendication 1 dérivé d'un glycopeptide du type 2-(2-acétamido-2-désoxy-3-O-D-glucopyranosyl)alcanoyl-aminoacyl-norLeucine sur lequel est greffé, par couplage covalent, au moins un groupe porteur d'au moins un site antigénique.

3. Composé du type 2-(2-acétamido-2-désoxy-3-O-D-glucopyranosyl)-alcanoyl-aminoacyl-norLeucine ou d'un dérivé de ce glycopeptide, ce composé étant caractérisé par la formule :

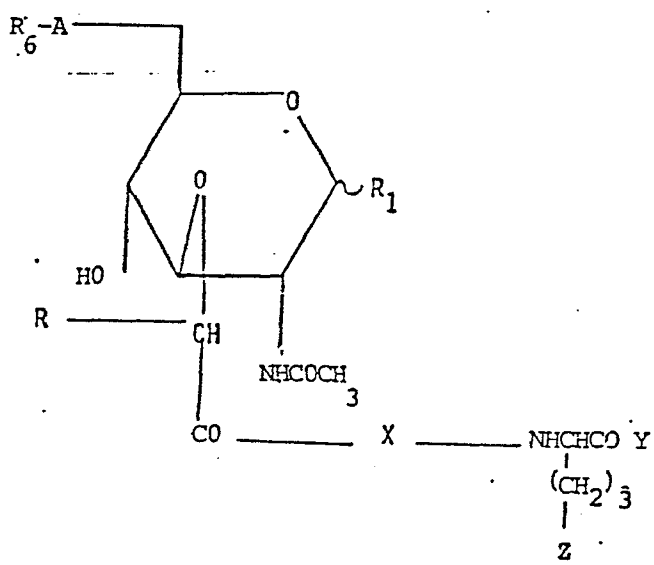

dans laquelle :

- $R_1$ est un groupe OH ou un groupe $O-C_6H_4-NH_2$ ou $O-(CH_2)_m-R_a$, m étant un nombre entier de 1 à 10 -, et $R_a$ étant une fonction amine, carboxyle, thiol, hydroxyle,
- A est de l'oxygène ou un groupe NH ;
- $R_6$ est de l'hydrogène ou un groupe B ou CO-B dans lequel B est un groupe ou une chaine linéaire ou ramifiée pouvant comporter jusqu'à environ 100 atomes de carbone, ce groupe B étant encore éventuellement porteur de un ou plusieurs

groupements fonctionnels, tels qu'amino, hydroxyle, carboxyle, carbonyle, acyle, acoxyle, cyclopropane,

- R est un hydrogène ou un groupe alcoyle comprenant de 1 à 4 atomes de carbone, notamment méthyl ;
- X est un résidu alanyl, arginyl, lysyl, asparagyl, aspartyl, cystéinyl, glutaminyl, glutamyl, glycyl, histidyl, hydroxyprolyl, isoleucyl, leucyl, méthionyl, phénylalanyl, prolyl, séryl, thréonyl, tryptophanyl, ou valyl ;
- Y est un groupe OH, $NH_2$, $R_7$,$OR_7$ ou $NHR_7$, $R_7$ étant un groupe hydrocarboné pouvant avoir de 1 à 20 atomes de carbone ou pouvant être un acide aminé tel qu'indiqué pour X, ledit acide aminé étant à son tour libre, amidé ou estérifié par un groupe d'estérification pouvant lui-même comporter jusqu'à 4 atomes de carbone;
- Z est un groupe alcoyle comprenant de 1 à 3 atomes de carbone, de préférence $-CH_3$ ou $-CH_2-CH_3$.

4. Composé selon la revendication 3, choisi parmi ceux qui ont des propriétés adjuvantes.

5. Composé selon la revendication 3 ou la revendication 4, ce composé ayant la formule selon la revendication 3 et dans laquelle $R_1$, A, $R_6$, R, X et Z ont les mêmes significations que dans la revendication 3 et dans laquelle Y est un groupe $R_7$,$OR_7$ ou $NHR_7$, $R_7$ étant un groupe hydrocarboné pouvant avoir de 1 à 10 atomes de carbone.

6. Composé selon l'une quelconque des revendications 3 à 5, ce composé ayant la formule de la revendication 3 dans laquelle A, R, X et Z sont tels que designés dans la revendication 3, Y est un groupe $R_7$, $OR_7$ ou $NHR_7$, $R_7$ étant tel que défini dans la revendication 3 ou dans la revendication 5, $R_6$ est l'hydrogène et $R_1$ est un groupe $O-(CH_2)_m-R_a$, m et $R_a$ étant tels que définis dans la revendication 3.

7. Composé selon la revendication 6, caractérisé en ce qu'il comporte un groupe porteur d'au moins un groupe

antigénique, greffé par couplage covalent sur le glycopeptide par l'intermédiaire d'un groupe antérieurement fonctionnel du groupe $R_a$.

8. Composé selon l'une quelconque des revendications 3 à 5, ce composé ayant la formule de la revendication 3 dans laquelle A, R, X et Z sont tels que définis dans la revendication 3, Y est un groupe $R_7$, $OR_7$ ou $NHR_7$, $R_7$ étant tel que défini dans la revendication 3 ou dans la revendication 5, $R_1$ est un groupe OH et $R_6$ est un groupe B ou CO-B, B étant tel que défini dans la revendication 3.

9. Composé selon la revendication 8, caractérisé en ce qu'il comporte un groupe porteur d'au moins un groupe antigénique greffé par couplage covalent sur le glycopeptide par l'intermédiaire d'un groupe antérieurement fonctionnel du groupe B.

10. Composé selon l'une quelconque des revendications 3 à 10, caractérisé en ce que Z est un groupe méthyle, le groupe

$$\begin{array}{l} NH-CH-COY \\ \quad\;\;\; | \\ \quad (CH_2)_3 \\ \quad\;\;\; | \\ \quad\;\;\; Z \end{array}$$

étant de préférence dérivé de la norLeucine.

11. Composé selon l'une quelconque des revendications 3 à 10, caractérisé en ce que, sauf glycyle, X est un acide aminé lévogyre.

12. Composé selon la revendication 11, caractérisé en ce que X est un groupe glycyle, valyle, notamment L-valyle ou séryle, notamment L-séryle.

13. Composé selon la revendication 11, caractérisé en ce que X est un groupe alanyle.

14. Compose selon l'une quelconque des revendications3 à 13, caractérisé en ce que Y est un groupe $R_7$,$OR_7$ dans lequel $R_7$ est un groupe alcoyle comportant de 1 à 10 atomes de carbone.

15. Composé selon la revendication 14, dans lequel $R_7$ est un groupe méthyle.

16. Composé selon la revendication 14, dans lequel $R_7$ est un groupe n-butyle.

17. Composé selon l'une quelconque des revendications 3 à 16, caractérisé en ce que R est un groupe méthyle.

18. Composé selon l'une quelconque des revendications 3 à 17, à l'exclusion de la revendication 7 ou de la revendication 9, caractérisé en ce que :

- $R_1$ est OH,
- A est de l'oxygène,
- $R_6$ est de l'hydrogène.

19. Composé selon l'une quelconque des revendications 3 à 17, caractérisé en ce que A est de l'oxygène et $R_6$ est un groupe $-CO-(CH_2)_n-COOH$, avec n étant un nombre de 1 à 10, notamment un groupe $-CO-(CH_2)_2-COOH$, ou un groupe $-CO-(CH_2)_n-NH_2$, avec n étant un nombre de 1 à 10, notamment un groupe $-CO-(CH_2)_6-NH_2$, le groupe terminal, amine ou carboxyle, de $R_6$, étant le cas échéant substitué par un groupe porteur d'au moins un site antigénique.

20. MurNAc-L-Ala-D-Nle-$NH_2$.

21. Composé caractérisé en ce qu'il est choisi parmi l'un des suivants :

MurNAc-L-Ala-D-Nle-On-$C_4H_9$
MurNAc-Gly-D-Nle-On-$C_4H_9$
MurNAc-L-Val-D-Nle-On-$C_4H_9$
<u>nor</u> MurNAc-L-Val-D-Nle-On$C_4H_9$
MurNAc-L-Ala-D-Nle-$OCH_3$
MurNAc-L-Ala-D-Nle-On$C_6H_{13}$.
MurNAc-L-Ala-D-Nle-$C_4H_9$

22. Composition, ayant notamment des propriétés immunoadjuvantes, caractérisée par l'association d'une dose efficace d'un composé immunoadjuvant conforme à l'une quelconque des revendications 1 à 21 avec un excipient physiologiquement acceptable.

23. Composition selon la revendication 22, caractérisée en ce qu'elle contient également un principe immunogène contre lequel une protection est recherchée chez l'hôte vivant auquel il est destiné.

24. Composition ayant des propriétés protectrices

*in vivo*, caractérisé par l'association avec un véhicule physiologiquement acceptable d'une dose efficace d'un composé comportant au moins un groupe porteur d'un site antigénique et conforme à l'une quelconque des revendications 3 à 17 ou à la revendication 19, ce groupe porteur du susdit site antigénique étant immunologiquement approprié à l'induction de la synthèse *in vivo* d'anticorps protecteurs contre ledit agent déterminé.

Office européen des brevets

**RAPPORT DE RECHERCHE EUROPEENNE**

0201420

Numéro de la demande

EP 86 40 0956

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | P.A. CADBY et al.: "Progress in the chemistry of organic natural products", vol. 40, 1981, pages 1-47, Springer-Verlag, Vienne, AT;<br>* Pages 9-13 *<br>----- | 1 | C 07 K 9/00<br>A 61 K 37/00<br>A 61 K 39/39 |
| | | | DOMAINES TECHNIQUES RECHERCHES (Int. Cl.4) |
| | | | C 07 K 9/00<br>A 61 K 37/00<br>A 61 K 39/00 |

Le présent rapport de recherche a été établi pour toutes les revendications

| Lieu de la recherche | Date d'achèvement de la recherche | Examinateur |
|---|---|---|
| LA HAYE | 01-07-1986 | VERHULST W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant